# EUROPEAN PATENT SPECIFICATION

(11) **EP 2 651 337 B1**
(45) Date of publication and mention of the grant of the patent: **23.08.2023**
(21) Application number: 11848038.3
(22) Date of filing: 14.12.2011
(51) Int. Cl.: A61F 2/24

(54) **PERCUTANEOUSLY DELIVERABLE HEART VALVE INCLUDING FOLDED MEMBRANE CUSPS WITH INTEGRAL LEAFLETS**
PERKUTAN VERABREICHBARE HERZKLAPPE MIT GEFALTETEN MEMBRANKLAPPENSEGELN MIT INTEGRIERTEN KLAPPEN
VALVULE CARDIAQUE APTE À ÊTRE POSÉE PAR VOIE PERCUTANÉE, COMPRENANT DES VALVES À MEMBRANES REPLIÉES À FEUILLETS INTÉGRÉS

(30) Priority: 14.12.2010 US 423051 P
(43) Date of publication of application: 23.10.2013
(73) Proprietor: Colibri Heart Valve LLC, Broomfield, CO 80020 (US)
(72) Inventor: FISH, David, R., Houston, TX 77005 (US); INDUNI, Eduardo, Alajuela, 906 4050 (CR); PANIAGUA, David, Houston, TX 77025 (US)
(74) Representative: Hoefer & Partner Patentanwälte mbB
(86) International application number: PCT/US2011/064989
(87) International publication number: WO 2012/082952

(56) References cited:
- WO-A2-2011/109450
- US-A- 5 509 930
- US-A1- 2007 043 431
- US-A1- 2007 043 431
- US-A1- 2009 112 309
- US-A1- 2009 112 309
- US-A1- 2010 185 277
- US-B1- 6 287 334

## Description

### FIELD

The present invention relates to the field of medical devices, and more particularly, to percutaneously deliverable heart valves.

### BACKGROUND

The native heart valves, and in particular, the aortic valve, has a complex geometry that endows both ideal opening and closing geometries through an anatomic joining of a tubular inflow structure of the left ventricular outflow tract and an expansion of the valve sinuses above the hinging point of the valve leaflets defined by the aortic valve annular ring, part of the fibrous "skeleton" of the heart.

For the purposes of discussion and definition in the ensuing descriptions, the "upper", downstream outlet structure of the native aortic valve above its hinging point contains three valve "cusps" of a generally spherical contour with central mobile portions termed "leaflets" that are induced by fluid pressure gradients to meet centrally to close and to move radially outward to open in valve operation. The cusps are further continuous with downstream curved tissue walls meeting the tubular great vessel, the aorta, at the "sino-tubular junction". Each cusp and its upper, downstream extension above the level of leaflet closure ("coaptation") are a continuous structure of a generally spherical contour and together define the envelope of the "sinus of Valsalva. Typically, surgical prosthetic valves are implanted by excision of the diseased native valve leaflets at the level of the annular ring, and suturing of the prosthetic valve at this point, thus replacing only the opening geometry of the valve and leaving the outer structures of the cusps and the sinuses of Valsalva, the anatomy that confers proper closing geometry, generally intact.

Surgical valve prostheses are generally constructed as analogs to this central portion of the native valve geometry involved in the opening phase of the valve cycle. This approach to modeling the replacement valve prosthesis is enabled by the nature of the surgical technique: the replacement valve is sutured into the valve seat under direct vision. In contrast, a percutaneous stent-mounted heart valve ("PHV") is typically a construct in which the operating valve membrane leaflets are mounted and confined within the tubular envelope of a collapsible frame for effective transvascular delivery.

Further, in order to preclude valve regurgitation, the base of each leaflet must lie in exact apposition to the valve seat to form a seal, a condition that is difficult to satisfy without implantation under direct vision. Even then, since the diseased native valve would not be removed and its axial geometry is often distorted, it may not be possible to seat a PHV exactly under any circumstances. Thus, a cylindrical cuff layer, interior or exterior to the frame, is usually employed that acts as a seal and provides some latitude in the positioning and alignment of the PHV along the axis of flow, allowing for reliable and effective PHV implantation and minimizing the risk of significant valve regurgitation. Finally, the diseased native valve leaflets, when pushed outward by the deployed PHV frame, may themselves form a barrier separating the sinuses of Valsalva from the leaflets of the PHV, then disrupting the native closing geometry of the valve so that the sinuses are no longer continuous with the pressurized space above the PHV leaflets.

These issues illustrate some of the challenges to the formation of a PHV; that is, how to confine operating leaflets within a partially sealed tubular structure while preserving ideal opening and closing valve behavior without the benefit of the natural mechanism of the sinuses of Valsalva in a single valve and leaflet geometry, such as the separate and distinct upper and lower geometries of the native valve. As such, there is a need for additional devices, systems and/or methods that address one or more of the problems or shortcomings noted above.

Further, document US 2009/112309 A1 discloses that a Catheter Based Heart Valve is described herein which replaces a non-functional, natural heart valve. The Catheter Based Heart Valve significantly reduces the invasiveness of the implantation procedure by being inserted with a catheter as opposed to open heart surgery. Additionally, the Catheter Based Heart Valve is coated with a biocompatible material to reduce the thrombogenic effects and to increase durability of the Catheter Based Heart Valve. The Catheter Based Heart Valve includes a stent and two or more polymer leaflets sewn to the stent. The stent is a wire assembly coated with Polystyrene-Polyisobutylene-Polystyrene. The leaflets are made from a polyester weave as a core material and are coated with Polystyrene-Polyisobutylene-Polystyrene before being sewn to the stent. Other biocompatible materials may be used, such as stainless steel, Titanium, Nickel-Titanium alloys, etc.

Document US 6 287 334 B1 discloses a valve prosthesis which is implantable within a vein or other blood vessel of a patient using a minimally-invasive surgical procedure. The prosthesis includes a tubular wire frame which presses radially outward against the inner walls of the blood vessel following implantation to hold the prosthesis in position. Multiple flow-resistive pockets that open and close in response to changes in blood flow direction are attached to the frame to impede the flow of blood in the reverse direction. The prosthesis is implanted using an introducer catheter which holds the prosthesis in a radially-compressed state as the prosthesis is inserted into and positioned within the blood vessel.

Document US 2007/043434 A1 discloses that biodegradable stent is comprised of a stereocomplex of polylactide enantiomers. The unique characteristics of stereocomplex polymers allow for the stent to have variable material characteristics along its length, while retaining uniform geometry. Thus, the stent is designed to be flexible upon insertion through the blood vessels while still retaining its rigidity and support upon expansion within the vessel. Furthermore, the material characteristics can be manipulated such that high strengths are possible even with small thicknesses of struts within the stent.

### SUMMARY

It is to be understood that the present invention includes a variety of different versions or embodiments, and this Summary is not meant to be limiting or all-inclusive. This Summary provides some general descriptions of some of the embodiments, but may also include some more specific descriptions of other embodiments.

Two goals of at least some embodiments of the present inventions are: (1) to maximize effective orifice area and minimize opening pressure gradients through geometry that mimics the natural form of inflow into the valve - the tubular outflow tract of the heart pumping chamber; and (2) to minimize the inward tension on the leaflet commissures in the closed position through geometry that mimics the natural effect of the sinuses of Valsalva - an effect that prevents downward displacement of the leaflet free edges under closing pressure, thus distributing force along the lines of leaflet apposition rather than focusing it at the points of leaflet attachment to the frame.

The first of these goals dictates that the inflow to the valve, similar to that of the natural aortic valve, encounters then outwardly displaces the most central portion of the leaflets first, with opening moving progressively outward along the surface of the leaflets. The second suggests that the cross-sectional profile of the valve sinus/cusp formed in its central portion by the free edge of the leaflets, like that of the natural aortic valve, should be approximately elliptical, and that the cross-sectional diameter of each cusp should progressively decrease below the plane of leaflet apposition, like that of the natural valve cusps. One or more embodiments of the one or more present inventions answer the configuration ideals with a robust balance of functional geometries for valve opening and closing.

The spherical geometry of the native aortic valve leaflets is difficult to replicate in a transcatheter valve. First, while this shape is functionally robust in vivo, even if reproduced in some form it is not suited to efficient radial compression typically required for collapse into a small diameter delivery catheter used in transcatheter valve delivery systems, and discontinuities would develop in the leaflet surface that would resolve into irregular folds with at least some circumferential component, thereby threatening the restitution of the geometry on reopening at deployment. Second, tissue bioprosthetic valve leaflets, if not actually constituted of the animal valve itself, are typically constructed of flat sheet tissue membrane from which rendering of cusps with leaflets of a spherical contour would be difficult if not impossible without the use of traction force on the material, or extensive cutting and suturing of the leaflet cusp portion - an impractical approach, and a threat to the material integrity of the thin tissue membrane.

The invention relates to a transcatheter, implantable prosthetic heart valve and is defined by the appended claims. At least one embodiment answers these challenges by employing conical rather than spherical cusp geometry, thereby reproducing some benefits of the latter with near-elliptical leaflet cross-section that progressively decreases moving proximal to the plane of leaflet apposition while being readily conformed on outward radial compression in the valve opening phase into a substantially flat folded construct against the interior tubular walls of the containing frame. This favorable resolution of the conical geometry in opening phase expresses the opening efficiency of this valve design with a large effective orifice area and low transvalvular energy losses. In the closed position, the free edges of the separate leaflets of the conical cusps meet in apposition, each cone acting as an independent valve; pressure load-bearing is enhanced by the material continuity of the cone structure with the inner apposing wall and outer wall of each cone being part of a single continuous membrane structure. Further, the conical cusps are particularly suited for compression and containment within a collapsible frame for transcatheter delivery.

In at least one embodiment, a transcatheter, percutaneously implantable, bioprosthetic heart valve having a lattice frame comprising a substantially tubular alloy metal mesh, and two or more valve cusps with leaflets mounted to the lattice frame, is provided. Further, the cusps include a flat sheet of processed mammalian tissue membrane that is folded into a substantially conical shape according to a flat folding pattern, the substantially conical shape is further formed by joining opposing sides of the substantially conical shape along a seam that is oriented along a longitudinal axis of the substantially conical shape. In at least one embodiment, the two or more cusps are attached along their seams (which may or may not include the apexes of the cusps), such as, by way of example and not limitation, along the axial centerline of the outer circumference of the cone, to an interior portion of the lattice frame along an axial flow direction of the valve and are further attached along the distal, downstream, edge of the substantially conical shape along at least an outer half of the substantially conical shape's edge. When the membrane valve leaflet is attached to the frame, its principal line of securement along the axial centerline of the outer circumference of the cone is attached at a non-commissural seam or edge, effecting a coaxial (to the flow axis) line of attachment at an area of the structure that advantageously bears load, thereby relieving the commissural attachment of loads associated with the securement of the cusp structures to the frame. As such, the leaflet commissure attachments, thus located at points where the leaflet membrane is continuous and uncut, advantageously need only bear the centripetal loads associated with the radially inward movement and operation of the free edges of the leaflets.

In at least one embodiment, a transcatheter, percutaneously implantable, bioprosthetic heart valve is provided wherein two distal, downstream, vertices of the flattened cusp and leaflet structure are folded over in a radially outward direction and fixed to the frame such that the vertex folds of neighboring leaflets are adjacent and define an extent of leaflet apposition at the points corresponding to leaflet commissures.

In at least one embodiment, a transcatheter, percutaneously implantable, bioprosthetic heart valve is provided wherein a vertex forming a proximal, upstream, apex of the substantially conical shape is folded over in a radially outward direction and affixed to an inner portion of the frame.

In at least one embodiment, a transcatheter, percutaneously implantable, bioprosthetic heart valve is provided wherein the flat folding pattern is polygonal and includes extending portions that, when the leaflet is mounted, extend circumferentially outward from an axial line of attachment of the leaflet to the frame so as to form, when joined and attached to corresponding extending portions of neighboring leaflets, an integral, inner, luminal, circumferentially partial or complete sealing cuff.

In at least one embodiment, a transcatheter, percutaneously implantable, bioprosthetic heart valve is provided wherein a separate tubular sealing cuff of tissue membrane is attached to an outer, abluminal surface of the frame to form a sealing cuff. In at least one embodiment, the membrane sheet is a single layer of a substantially homogenous material. In at least one embodiment, the membrane sheet is an unlaminated single layer of material. In at least one embodiment, the membrane sheet is a single layer of material that does not include any reinforcement, such as reinforcing fibers. In at least one embodiment, the membrane sheet is a single layer of treated pericardium tissue. In at least one embodiment, the membrane sheet is a single layer of a synthetic film.

Various components are referred to herein as "operably associated." As used herein, "operably associated" refers to components that are linked together in operable fashion, and encompasses embodiments in which components are linked directly, as well as embodiments in which additional components are placed between the two linked components.

As used herein, "at least one," "one or more," and "and/or" are open-ended expressions that are both conjunctive and disjunctive in operation. For example, each of the expressions "at least one of A, B and C," "at least one of A, B, or C," "one or more of A, B, and C," "one or more of A, B, or C" and "A, B, and/or C" means A alone, B alone, C alone, A and B together, A and C together, B and C together, or A, B and C together.

The invention is defined by the independent claim. The dependent claims contain advantageous embodiments of the present invention.

### BRIEF DESCRIPTION OF THE DRAWINGS

To further clarify the above and other advantages and features, a more particular description is rendered by reference to specific embodiments which are illustrated in the appended drawings. It should be appreciated that these drawings depict only typical embodiments.

The embodiments are described and explained with additional specificity and detail through the use of the accompanying drawings in which:
Fig. 1A is a plan view of a flat sheet membrane template for the formation of an integrated cusp and leaflet folded structure in accordance with at least one embodiment of the present invention;
Fig. 1B is an oblique axial top (distal) perspective view directed downward (proximal) and radially outward of a folded membrane sheet after execution of the template foldings illustrated in Fig. 1A, thereby yielding a completed integrated cusp and leaflet folded structure;
Fig. 1C is a side perspective view directed radially outward of the inner aspect of an initially folded version of the integrated cusp and leaflet template shown in Fig. 1A;
Fig. 1D is an oblique axial top (distal) perspective view directed downward (proximal) and radially outward of a further partially folded version of the integrated cusp and leaflet folded structure shown in Fig. 1C;
Fig. 1E is an another oblique axial top (distal) perspective view directed downward (proximal) and radially outward of a further partially folded version of the integrated cusp and leaflet folded structure shown in Fig. 1D;
Fig. 1F is a modified version of the integrated cusp and leaflet folded structure shown in Fig. 1E;
Fig. 1G is same structure and view shown in Fig. 1E, along with a top (distal) cross-section schematic view of the distal end of a three-leaflet valve in a closed operating position;
Fig. 2 is a plan view of another flat sheet membrane template for the formation of an integrated cusp and leaflet folded structure in accordance with at least one embodiment of the present invention;
Fig. 3 is a plan view of yet another flat sheet membrane template for the formation of an integrated cusp and leaflet folded structure in accordance with at least one embodiment of the present invention;
Fig. 4 is a plan view of still yet another flat sheet membrane template for the formation of an integrated cusp and leaflet folded structure in accordance with at least one embodiment of the present invention;
Fig. 5A is a plan view of another flat sheet membrane template for the formation of an integrated cusp and leaflet folded structure in accordance with at least one embodiment of the present invention;
Fig. 5B is an oblique axial top (distal) perspective view directed downward (proximal) and radially outward of a partially folded version of an integrated cusp and leaflet folded structure prepared in accordance with the template shown in Fig. 5A;
Fig. 5C is an oblique axial top (distal) perspective view directed downward (proximal) and radially outward of a further partially folded version of the integrated cusp and leaflet folded structure shown in Fig. 5B;
Fig. 5D is plan view of the inner (luminal) aspect of a completely folded version of the structure of Fig. 5C, thereby yielding a completed integrated cusp and leaflet folded structure prepared in accordance with the template shown in Fig. 5A (with the exception of unfolded commissure tabs);
Fig. 5E shows a detail perspective view of a folded commissure tab;
Fig. 5F shows a perspective view of the outer (abluminal) aspect of the device shown in Fig. 5D;
Fig. 6 is a plan view of yet another flat sheet membrane template for the formation of an integrated cusp and leaflet folded structure in accordance with at least one embodiment of the present invention;
Fig. 7A is a plan view of still yet another flat sheet membrane template for the formation of an integrated cusp and leaflet folded structure in accordance with at least one embodiment of the present invention;
Fig. 7B is an oblique axial top (distal) perspective view directed downward (proximal) and radially outward of a partially folded version of an integrated cusp and leaflet folded structure prepared in accordance with the template shown in Fig. 7A;
Fig. 7C is an oblique axial top (distal) perspective view directed downward (proximal) and radially outward of a further partially folded version of the integrated cusp and leaflet folded structure shown in Fig. 7B;
Fig. 7D is an oblique axial top (distal) perspective view directed downward (proximal) and radially outward of yet a further partially folded version of the integrated cusp and leaflet folded structure shown in Fig. 7C;
Fig. 7E shows a shallow oblique top perspective view of the outer (abluminal) aspect of the partially folded cusp and leaflet structure of Fig. 7D;
Fig. 7F is a plan view of the inner (luminal) aspect of a completely folded version of the structure of Fig. 7D yielding an integrated cusp and leaflet folded structure prepared in accordance with the template shown in Fig. 7A (excepting that the commissure tabs and apex are not yet folded outward);
Fig. 7G is a side perspective view of the outer (abluminal) aspect of the structure of Fig. 7F showing a completely folded version of an integrated cusp and leaflet folded structure prepared in accordance with the template shown in Fig. 7A (excepting that the commissure tabs and apex are not yet folded outward);
Fig. 7H is a plan view of the inner (luminal) aspect of a completely folded version of an integrated cusp and leaflet folded structure prepared in accordance with the template shown in Fig. 7A;
Fig. 7I is a plan view of the outer (abluminal) aspect of a completely folded version of an integrated cusp and leaflet folded structure prepared in accordance with the template shown in Fig. 7A;
Fig. 7J is an oblique top (distal) perspective view of a completely folded version of an integrated cusp and leaflet folded structure prepared in accordance with the template shown in Fig. 7A;
Fig. 7K is a top perspective view directed downward (proximal) into the cusp space of an integrated cusp and leaflet folded structure prepared in accordance with the template shown in Fig. 7A;
Fig. 8A is an oblique top (distal) perspective view of an embodiment of a lattice frame for mounting three of the single-piece folded integrated cusp and leaflet structures as described herein;
Fig. 8B is a side elevation view of the lattice frame shown in Fig. 8A;
Fig. 8C is a side elevation view of the lattice frame of Fig. 8A with a superimposed plan view of the radially outer aspect of the completely folded integrated cusp and leaflet structure of Fig. 7I;
Fig. 8D is an oblique axial (top/distal) perspective view of an assembled three-leaflet valve in accordance with at least one embodiment;
Figs. 9A and 9B are two different oblique axial (top/distal) perspective views of another embodiment of a lattice frame for mounting three of the single-piece folded integrated cusp and leaflet structures that include commissure tabs;
Fig. 9C is a side perspective view of the lattice frame shown in Figs. 9A and 9B with a superimposed plan view of the outer aspect of the completely folded integrated cusp and leaflet structure of Fig. 7I;
Fig. 9D is a side view of the lattice frame shown in Figs. 9A and 9B with superimposed views of the outer aspect of two circumferentially adjacent completely folded integrated cusp and leaflet structures; and
Fig. 9E is an oblique axial (top/distal) perspective view of an assembled three-leaflet valve comprising the lattice frame shown in Figs. 9A and 9B and three identical folded integrated cusp and leaflet structures.

The drawings are not necessarily to scale.

### DETAILED DESCRIPTION

One or more embodiments of the invention described herein include an implantable prosthetic heart valve having a frame and two or more cusp and leaflet structures mounted to the frame. The frame preferably comprises a lattice of substantially tubular alloy metal mesh. The cusp and leaflet structures include a membrane operable to open and close, thereby providing a functioning valve when mounted within a frame. In at least one embodiment, the membrane preferably comprises a flat sheet of processed mammalian tissue membrane that is folded into a substantially conical shape according to a flat folding pattern.

In the ensuing descriptions and referenced figures it will be seen that, when applied to a dry sheet membrane, the folding initially results in a cusp shape of an inverted pyramid with a rhomboid base. On relaxation of the folds as occurs naturally with a flexible and pliable membrane, especially when the membrane is hydrated, the cusp shape becomes substantially conical in shape and will be described as such in the ensuing descriptions as it more closely represents the embodiment of the cusp in operation of the valve.

Formation of a valve construct as described herein provides a percutaneously deliverable heart valve with a relatively small diameter for transcatheter placement. That is, the substantially conical shape associated with the flat folding patterns used to form leaflets as described herein allow for construction of a valve that can be compressed prior to introduction to a catheter to an advantageously small diameter, thereby facilitating transcatheter percutaneous delivery of the valve within a patient. The substantially conical shape is further formed by joining two axially oriented sides of the substantially conical shape along a seam that is oriented along a longitudinal axis of the substantially conical shape. The two or more integrated cusp and leaflet structures are affixed to an interior portion of the lattice frame along an axial flow direction of the valve and are further affixed along the distal, downstream, edge of the substantially conical shape along at least an outer half of the substantially conical shape's edge.

One or more of the various embodiments described herein have a number of different features and characteristics as compared to other commercially available prosthetic heart valves. For example, at least one embodiment of a transcatheter, percutaneously implantable, prosthetic heart valve described below comprises a flat polygonal sheet membrane having more than four sides and which forms an integrated cusp and leaflet structure.

In addition, at least one embodiment of a transcatheter, percutaneously implantable, prosthetic heart valve described below comprises integrated cusp and leaflet structures that are attached to a lattice frame at the circumferential perimeter locations corresponding to the commissures. At such locations, the length of the seam that forms the common line of attachment of the cusp and integral leaflet to the frame is less than one-half to two-thirds of the axial length of the membrane portion of the valve.

In at least one embodiment of a transcatheter, percutaneously implantable, prosthetic heart valve, when the valve is in the open position, the mobile leaflet layer apposes or is geometrically free to appose its full outward surface completely to the immediately radially located outward structure, such as at least one of the cusp wall layer or interior surface of the lattice frame. In at least one embodiment, in the closed position the transverse cross-sectional length of the mobile leaflet layer and the cross-sectional area of the cusp/sinus space decreases monotonically from the distal end to proximal end of the membrane portion of the valve. (That is, generally the property of a cone as well as an inverted pyramid.)

In at least one embodiment, the mobile leaflet layer and the immediately outward structure for the full axial length of the leaflet (cusp wall layer, frame, or other) are a single continuous piece of material.

In at least one embodiment, at the base of each cusp (that is, at the most proximal extent of the leaflet), the circumferential extent of attachment of the membrane to the frame is less than the circumferential extent of attachment of the membrane to the frame at the distal end of the cusp. In addition, at the base of each cusp, the circumferential extent of transverse (that is, on a line or on the plane of a circumferential single-plane curve of folding that is generally perpendicular to the flow axis of the valve) folding of the membrane to the frame is less than the circumferential extent of transverse folding at the distal end of the cusp.

At least one embodiment, a prosthetic valve described herein comprises an integrated cusp and leaflet structure wherein the apposing sides of the cusp are joined at one or more axially oriented seams. In at least one embodiment, all folds and seams are located on line segments.

At least one embodiment of the one or more present invention does not include frame elements, such as support members, spanning the interior of the valve luminal to support one or more portions of the membrane sheet. Moreover, at least one embodiment of the invention does not include any hardware shaping form inward of or attached to any portion of the mobile leaflet portion of the membrane.

In addition, at least one embodiment of the invention does not utilize attachment of the leaflet layer to the frame along the substantially complete circumferential distance separating the commissures at any point below (more proximal than) the commissure tabs.

At least one embodiment of the invention does not include a transverse fold or reflection of the leaflet layer along the substantially complete circumferential distance separating the commissures at any point below (more proximal than) the commissure tabs.

### Nomenclature

For all embodiments presented herein it is to be understood that a "membrane" includes suitable materials for forming the cusps and leaflets. Accordingly, with regard to particular material types that may be used to form the membrane sheet, in at least one embodiment the membrane sheet forming the cusp or leaflet portions includes a one-piece, single layer sheet of biocompatible membrane, such as fixed mammalian pericardium tissue or synthetic biocompatible material, such as ePTFE. In at least one embodiment, the membrane sheet is made from a tissue preparation process that yields a leaflet material of suitable strength and durability for use in a prosthetic transcatheter deliverable heart valve. The content of WO 2011/109450A2 published on September 9, 2011. Although the membrane sheet is preferably a single piece of material, a membrane sheet formed of a plurality of pieces of material may be used, such as two to fifty or more pieces of material that are connected.

As used herein "proximal" means situated near or closer to the upstream or flow inlet end of the valve, and "distal" means situated near or closer to the downstream or flow outlet end of the valve. This convention is further applied in the description of the various folded structure elements (membrane sections, edge segments and fold lines) that are termed "proximal" or "distal" if the final position or orientation of said element within the completed folded structure satisfies the above definitions. Likewise, one of said elements is termed, "axial", "transverse" or "circumferential" to describe its position and orientation in the completed valve.

As used herein, a "cusp" means that structural portion of a valve related to a single leaflet that encompasses a space closed toward the lower (proximal) direction and open to the upper (distal) direction, formed by the joined and/or continuous structures of the mobile leaflet portion on the radially inner side and the cusp wall portion on the radially outer side. The "cusp" in the present invention is that structure described as having a substantially conical shape.

As used herein, the "mobile leaflet layer" or "leaflet" means that radially inward portion of the cusp that moves during operation of the valve. For example, when the valve is closing the mobile leaflet layer moves radially inward toward the central axis of the valve lumen. When the valve is opening, the mobile leaflet layer moves radially outward and away from the central axis of the valve lumen.

As used herein, the "cusp wall layer" means a portion of the cusp that resides radially outward of the mobile leaflet layer. In some embodiments, a portion of the cusp wall layer moves during operation of the valve. In other embodiments, the cusp wall layer remains substantially immobile during operation of the valve.

As used herein, the "cuff wall layer" means a portion of the folded membrane structure that resides radially outward of both the cusp wall layer and the mobile leaflet layer, and where present, is radially closest to the frame of the three layers comprising the mobile leaflet layer, the cusp wall layer, and the cuff wall layer. The cuff wall layer remains substantially immobile during operation of the valve.

A "frame" as used herein means a substantially tubular member that holds a plurality of cusps and/or leaflets. By way of example, the frame may be a wire lattice or a lattice cut from a single tubular piece of metal alloy, that is both collapsible and expandable.

A "valve" as used herein means a frame with a plurality of cusps and/or leaflets attached thereto. In the present invention each of said leaflets is an integral part of a folded membrane cusp structure. If a frame is used that is a metal lattice that is both collapsible and expandable, such a construct may be delivered through a catheter percutaneously to a target site within a patient, such as the aortic valve.

As used herein, "cone" or "conical" means resembling a cone or portion thereof at some point in the practical use of the structure.

As used herein "substantially conical" means resembling a cone or a portion thereof at some point in the practical use of the structure with the specific property that the transverse (that is, on a plane of section generally perpendicular to the axis of flow of the valve) cross-sectional perimeter or area of said structure in the operationally closed position decreases monotonically moving from the level of the leaflet apposition to the proximal end of the valve.

As used herein, "two or more leaflets," "two or more valve leaflets," "a plurality of leaflets" or a similar term means two, three, four, or more valve leaflets. Accordingly, "a valve with two or more leaflets" includes a valve with two leaflets, a valve with three leaflets, a valve with four leaflets, and a valve with more than four leaflets.

As used herein, a "folding" means the partition of a flat sheet section of material along a sharp line of folding or crease into subsections each lying on separate planes, but without interruption of material continuity.

As used herein, a "complete folding" means folding (as above) wherein the angular change of the planar axis at the line of folding is approximately 180 degrees, such that the subsections lie on approximately parallel planes and the subsections are in approximate overlying contact with each other at least at some point.

As used herein, a "cuff" means that portion of a valve structure that lies radially outward of the cusp wall portion that in some part circumferentially encompasses at least a portion of the cusp structure and acts to limit flow that may pass retrograde around the cusp.

As used herein, "commissure" means the site of union or junction between adjacent cusps and/or leaflets, and by extension, collectively those portions of the adjacent integrated cusp and leaflet structures that are coincident at the union or junction in the completed valve structure.

As used herein, an "integrated cusp and leaflet folded structure" means a membrane folded in accordance with one of the patterns described herein.

### Folded Valve Integrated Cusp and Leaflet - Folding Pattern No. 1

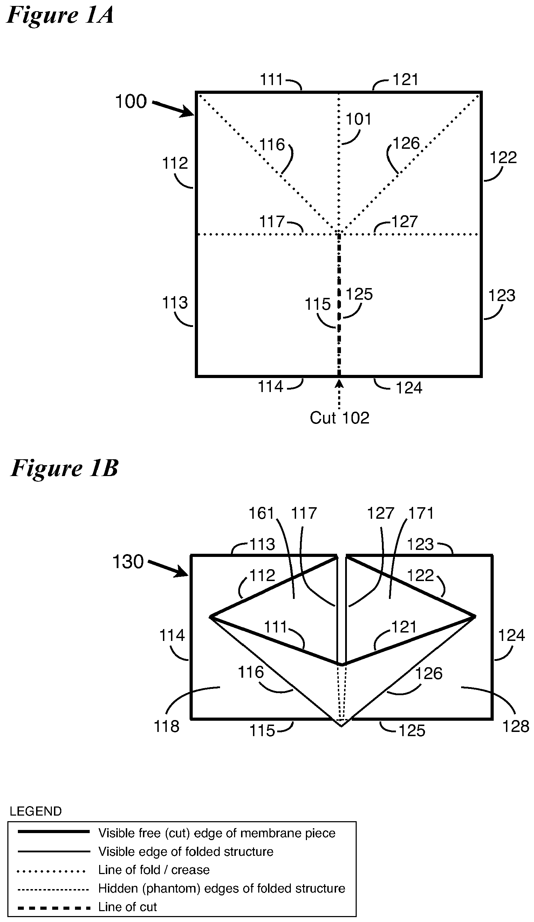

Referring generally to Figs. 1A-7K, each cusp embodiment of an integrated cusp and leaflet structure described herein is a substantially flattened cone collapsed along an axis substantially perpendicular to its longitudinal axis. In one or more embodiments, the integrated cusp and leaflet structure, when being formed from a piece of membrane, is readily realized by folding a flat sheet of membrane from a closed polygon pattern. The pattern folding results in apposing seam lines aligned along their axial length. These are joined to close the cusp in the general shape of a cone with the joined seam forming the "spine" along which the cusp meets the inner aspect of the tubular frame. It can be seen that, when formed of a dry sheet membrane, the pattern results initially in a cusp shape that is an inverted pyramid with a rhomboid base that, with a flexible, pliable membrane, is congruent to a substantially conical shape. On relaxation of the folds in practical use a substantially conical cusp is realized wherein the inner mobile operating portions of the leaflet are continuous with the outer portion that forms the integral wall of the cusp sinus or pocket.

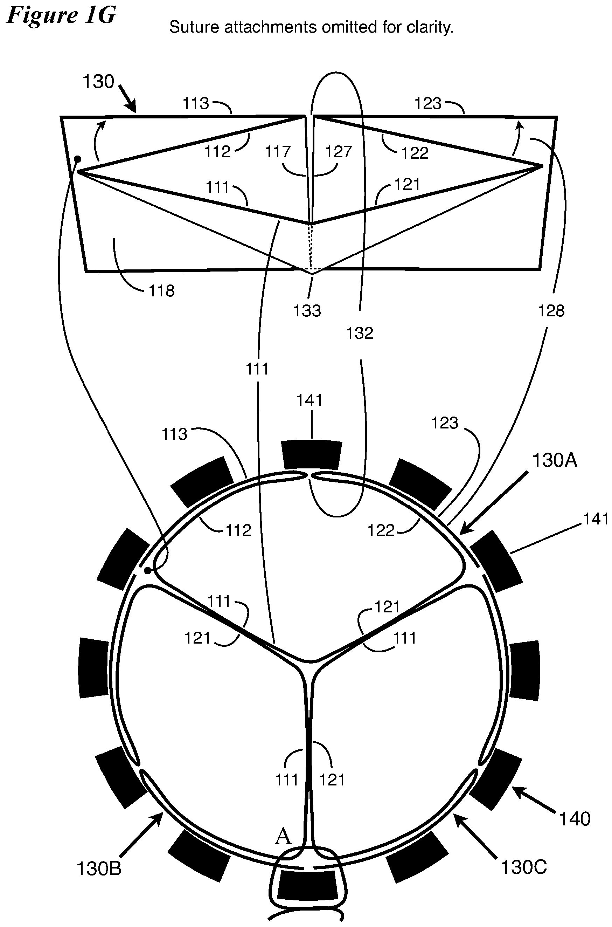

Referring now to Fig. 1A, a plan view of a rectangular flat sheet membrane template 100 is shown for the formation of a single-piece folded valve integrated cusp and leaflet. The plan view is shown with a view of that leaflet surface that faces radially inward once folded and mounted within a frame. Reference is also made to Fig. 1G, wherein a schematic of a valve in distal axial view is shown, and wherein three cusps with integral leaflets are shown within the frame that collectively form the valve. As described and illustrated in the present application, alternate polygons and other closed shapes may be employed with alternate folding patterns to generate alternate shapes and functional features of the valve cusp and leaflet, and complete valve.

Referring again to Fig. 1A, and in accordance with at least one embodiment of the one or more present inventions, dotted lines 101, 116, 117, 126 and 127 represent the position of folds or creases applied to a piece of membrane to form a leaflet structure 130. More particularly, folding at lines 116, 126 and 101 is initiated inward (with convexity of the surface disposed radially inward toward the central axis of the valve lumen) while folds 117 and 127 are folded initially outward (with convexity of surface disposed radially outward away from the central axis of the valve lumen). Since folding causes re-orientation of the various sections of the sheet template in relation to each other and to the valve geometry, final orientation of the fold lines within the structure on mounting and operation of the leaflets will not necessarily retain the same orientations as on initiation of the folds. The "inward" and "outward" conventions by this definition will be followed throughout the descriptions of the various folded geometries presented herein.

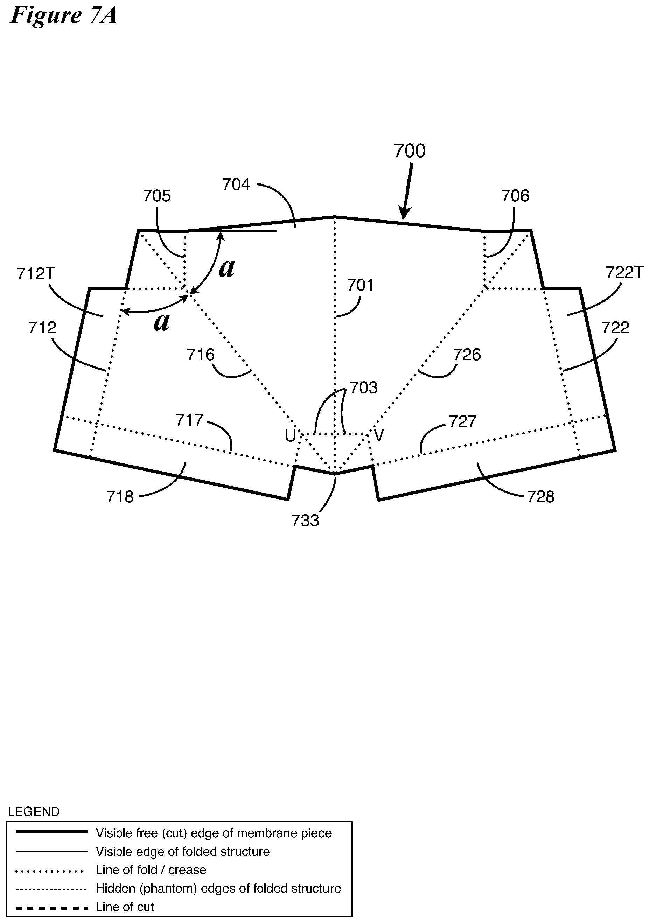

Referring again to Fig. 1A, a line of division by cutting is indicated at 102. Cutting at 102 results in opposing edges 115 and 125 that will be separated by folding. The other free edges of the structure are labeled as their position and orientation changes through the folding steps. Fold 101 defines the central axis of symmetry of the leaflet pattern, with the concave side of fold 101 facing radially outward toward the frame and away from the central axis of the valve lumen. Fold 101 assists in the maintenance of axial symmetry of the folded construct, but is not necessary to leaflet function and is not retained in the final operational form of the valve. (See Fig. 7A.)

Referring now to Fig. 1B, an oblique axial top (distal) perspective view of a substantially completed folded leaflet structure 130 is shown. (Three completed folded cusp and leaflet structures 130 are typically mounted to a frame to form an operating heart valve.)

The view of Fig. 1B is directed downward (proximally) and radially outward, with such view illustrating a substantially completed folded leaflet and cusp structure 130 that depicts the reoriented segments and sections of Fig. 1A after execution of the template foldings. Segments 111 and 121 form the left and right halves of the distal free edge of the mobile operating portion of the leaflet. Inward folding at 116 and 126 forms a second layer of membrane outward of the first, with segments 112 and 122 forming the distal free margin of the outer wall of the integrated cusp. In radially flatted form of the integrated cusp and leaflet structure (that is, approximating the open operating position of the leaflet), the segment 111 will appose to 112, and 121 will appose to 122.

The left cusp wall section 161 is bounded by folds 116 and 117 and edge segment 112. The right cusp wall section 171 is bounded by folds 126 and 127 and edge segment 122.

The left cuff wall section 118 is bounded by fold 117 and edge segments 113, 114 and 115. The right cuff wall section 128 is bounded by fold 127 and edge segments 123, 124 and 125. Inward folding at 117 and 127 cause these cuff wall sections 118 and 128 to position outward of the cusp wall sections 161 and 171, respectively. In radially flatted form of the completed folded structure (again, approximating the open operating position of the leaflet), the edge segment 113 will appose to 112, and edge segment 123 will appose to 122.

### Folded Valve Folding Sequence

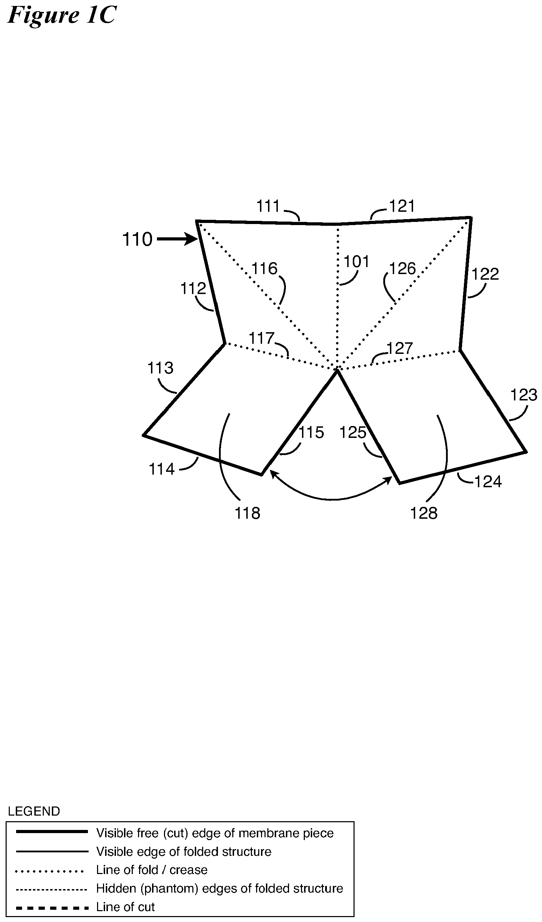

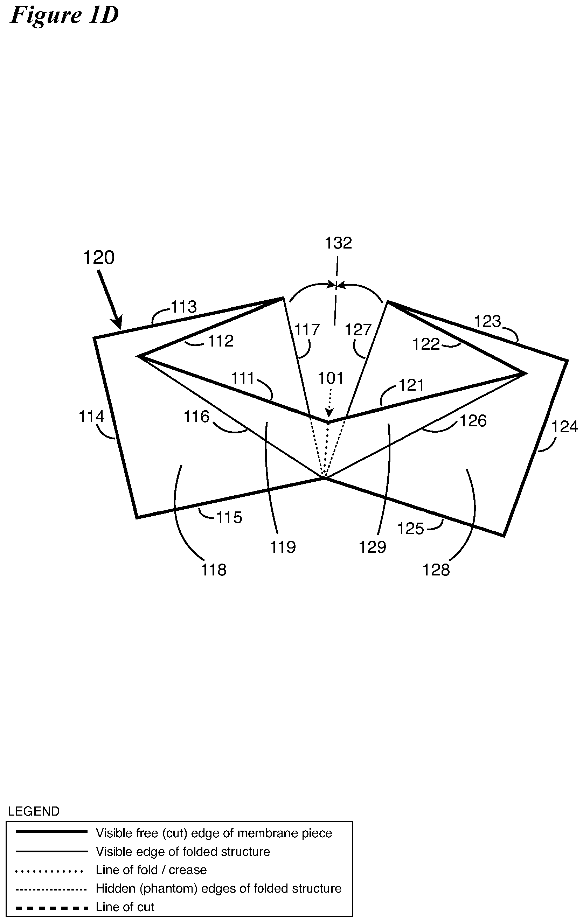

Referring now to Figs. 1C and 1D, oblique axial top (distal) perspective views of a partially completed folded leaflet and cusp are shown. The views provided by Figs. 1C and 1D are directed downward (proximally) and radially outward, with such views depicting the reoriented segments and sections of Fig. 1A after partial execution of the template foldings. Fig. 1C shows a perspective view of the inner aspect of the template 100 after initiation of the foldings and cutting at 102 resulting in left and right cuff wall sections 118 and 128, respectively. The cut free edges 115 and 125 are separated along with the left and right cuff wall sections 118 and 128 by outward folding at 117 and 127, respectively. Completed folding at 117 and 127 results in the cuff wall sections 118 and 128, respectively. Distally situated (with respect to the blood flow direction) edge segments 113 and 123 of the cuff wall sections 118 and 128, as well as proximally situated edge segments 115 and 125 of the cuff wall sections 118 and 128, are positioned transverse, and in at least one embodiment, substantially perpendicular, to the central axis of the valve.

Fig. 1D shows the cusp and leaflet structure 120 with the folds 116, 126, 117 and 127 at an intermediate stage of completion. Triangular left and right mobile leaflet sections 119 and 129 respectively are bounded by folds 101 and 116 and free edge segment 111 on the left, and folds 101 and 126 and free edge segment 121 on the right. Folds 117 and 127 are then brought into apposition on the outward aspect of the integrated cusp and leaflet along a seam line 132 where the folds will be joined and attached to a frame to close the shape of the single-piece continuous conical integrated cusp and leaflet.

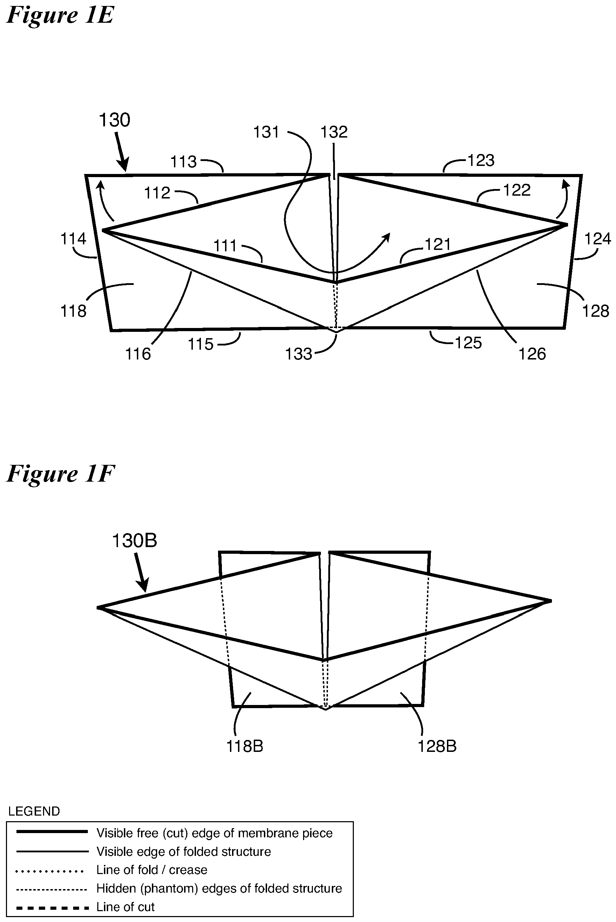

Referring now to Figs. 1E and 1F, oblique axial top (distal) perspective views of a substantially completed folded cusp and leaflet are shown. The views provided by Figs. 1E and 1F are directed downward (proximally) and radially outward, with such views depicting the reoriented segments and sections of Fig. 1A after execution of the template foldings.

Fig. 1E shows the cusp and leaflet folding substantially completed forming the structure 130 with the seam 132 formed by the apposition of folds 117 and 127, thus forming a generally conical cusp and sinus space 131. The triangular corners formed at the distal ends of folds 116 and 126 are apposed to and attached to the cuff wall sections 118 and 128, respectively. Between adjacent cusp and leaflet structures in a multi-leaflet valve, the folded corners form the junction joining the adjacent free edges (121 of leaflet A to 111 of leaflet B, for example) of the mobile leaflet portions. When further attached to the circumferential valve frame, these corners tether the free edges of the mobile leaflet portions to the circumferential inner boundary of the generally cylindrical valve frame, thus forming valve leaflet commissures at each similar join.

Referring now to Fig. 1F, a structure similar to that of Fig. 1E is depicted, but with the cuff wall sections 118B and 128B reduced in circumferential extent from that of leaflet structure 130 shown in Fig. 1E. More particularly, depending on the clinical application of the valve, a fully circumferential cuff wall may be unnecessary, and a valve with a limited cuff wall with less tissue membrane mass may offer functional advantages. Alternatively, an additional piece of membrane may be placed circumferentially around the outer abluminal surface of the valve frame to act as a sealing cuff to form a barrier against valvular regurgitation.

Referring again to Fig. 1E, the apex 133 (proximal tip) of the conical cusp and leaflet forms the lower (proximal) end of the seam 132. In at least one embodiment, the apex 133 is also attached to the circumferential boundary of the valve and valve frame.

Referring now to Fig. 1G, for ease of reference the structure of Fig. 1E is again shown in Fig. 1G at the top of the page, along with a top (distal) cross-section view of the distal end of a three-leaflet valve in the closed operating position. The three cusps with leaflets are shown residing within a lattice frame in order to indicate the configuration of elements between the folded integrated cusp and leaflet structure 130 and its disposition within a three-leaflet frame-mounted valve. Suture attachments are omitted for clarity.

For each folded integrated cusp and leaflet structure, the outer axial seam 132 is aligned with one or more frame members 141 in a manner to permit the attachment of the folds 117 to 127, and to the coincident frame member by the same attachment, for example, by a single knot or line of suture. Advantageously for this purpose, the frame may preferentially contain axially oriented members that align to the seam 132 for part or all of the full axial extent of the valve. Further, said axially oriented members may advantageously contain holes or notches for securing and tying suture.

In Fig. 1G at point A, an illustrated loop symbolizing a suture knot is shown to demonstrate that a single knot may advantageously pass through or engage the frame member and the six layers; that is, the mobile leaflet section, the cusp wall section, and the cuff wall section of each adjoining cusp and leaflet structure that are coincident at this site of the commissure.

Referring still to Fig. 1G, it can be seen that the folded integrated cusp and leaflet structure, when mounted within the lattice frame and placed in the closed operating position, manifests the following configurations: (1) the left leaflet free edge segment 111 is in each case apposed to the right leaflet free edge segment 121 of the adjacent leaflet; (2) the portions of the leaflets just proximal to the free edges, thus, are also apposed to form the contact seal that enables effective closing operation, thereby preventing valvular regurgitation; and (3) the distal edges 112 and 122 of the cusp wall sections are apposed to the distal edges of the cuff wall sections 113 and 123, respectively.

### Folded Valve Pattern Variation No. 2

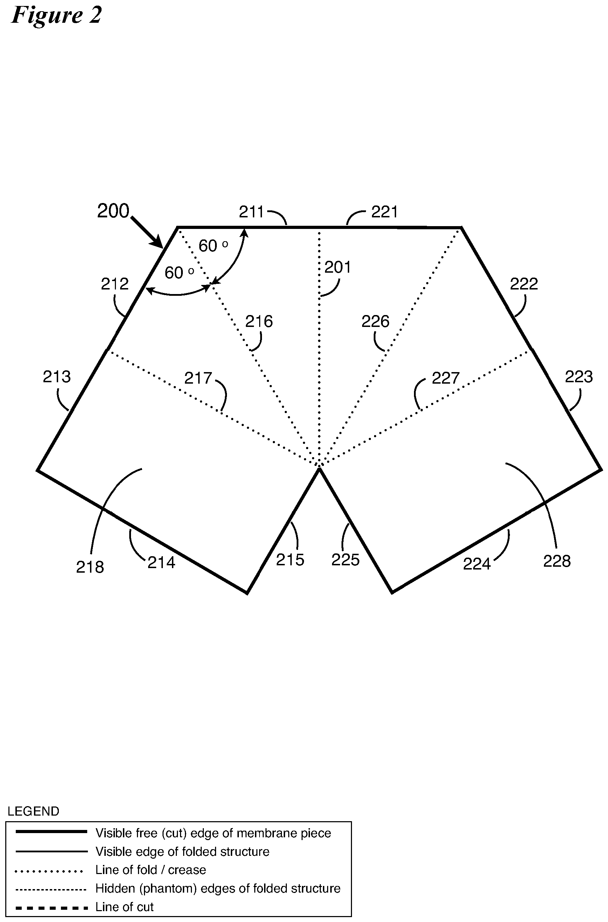

Referring now to Fig. 2, and in accordance with at least one embodiment, a plan view of a flat sheet membrane template 200 that is polygonal rather than rectangular is shown. Template 200 contains folds 201, 216, 226, 217 and 227 that correspond to folds 101, 116, 126, 117 and 127, respectively, and are disposed in like manner in folding execution, as are the segments enumerated. The folding pattern is designed to form a longer cone of the same diameter, which achieves a more distally disposed central point of valve leaflet coaptation, the mechanics of which are more tolerant of pressure loads. The pattern dimensions may be altered to suit the particular clinical application of the valve. The template examples disclosed herein are for enablement purposes and shall not be interpreted as limiting the scope of the claims. The example is shown for a cusp cone wall disposed at about a 60 degree angle to the horizontal (short axis) of the generally cylindrical valve geometry, whereas that angle for the rectangular pattern of Figs. 1A-1G was about 45 degrees.

### Folded Valve Pattern Variation No. 3

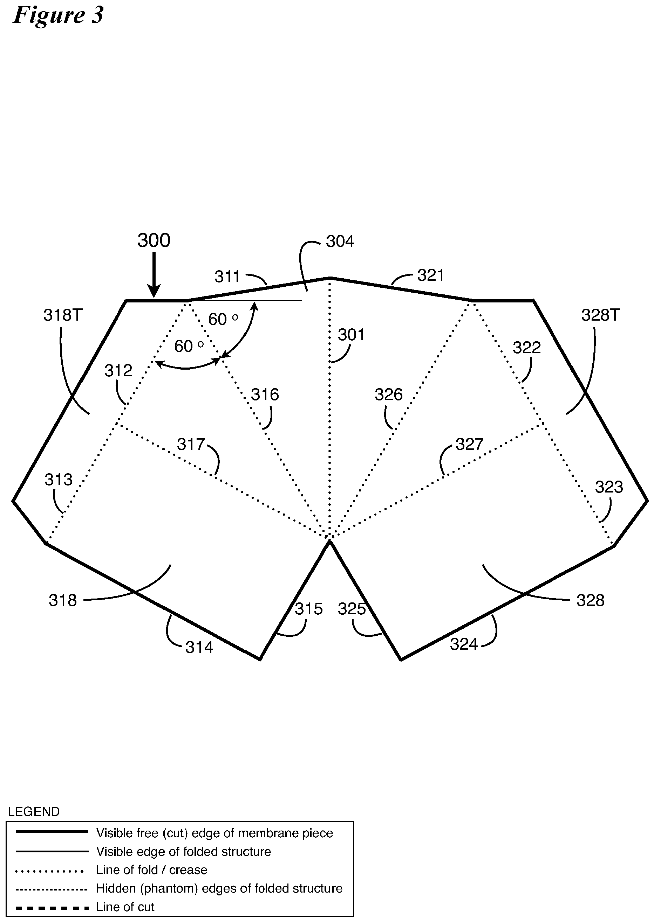

Referring now to Fig. 3, and in accordance with at least one embodiment, a plan view of template 300 is shown for a flat sheet membrane that contains the pattern 200 of Fig. 2 with added sections that extend the distal contour of the structure when completed in folding. More particularly, the free edge of the mobile leaflet section is extended distally with a section having a polygonal or curved free edge in order to increase the contacting area of leaflet apposition in valve closing operation. Additionally, the distal contour of the cusp wall sections and cuff wall sections 318 and 328 are extended by "tab" sections 318T and 328T, respectively. These added "tab" extensions allow for increased area by which to mount the outer wall of the cusp and leaflet assembly to the frame and for elevating the cuff wall "above" (more distal to) the plane of leaflet apposition, thereby also increasing the effective volume of the cusp in closing operation. These "tab" extensions, being distally disposed after completion of folding and initial mounting within the lattice frame, or a distal portion of them may optionally be folded radially outward along 312-313 and 322-323, for example, to wrap around the distal edge of the frame such that the "tab" extension areas 318T and 328T lie on the outer, abluminal aspect of the frame where, when attached to the frame, they potentially increase the strength of the cusp attachment.

Referring still to Fig. 3, template 300 contains folds 301, 316, 326, 317 and 327 that correspond to folds 101, 116, 126, 117 and 127, respectively, and are disposed in like manner in folding execution, as are the edge segments similarly enumerated. In addition to the tab features discussed in the preceding paragraph, as with template 200, template 300 is designed to form a longer cone of the same diameter, which achieves a more distally disposed central point of valve leaflet coaptation. Again, the pattern dimensions may be altered to suit the particular clinical application of the valve. The example is shown for a cusp cone wall disposed at about a 60 degree angle to the horizontal (short axis) of the generally cylindrical valve geometry.

### Folded Valve Pattern Variation No. 4

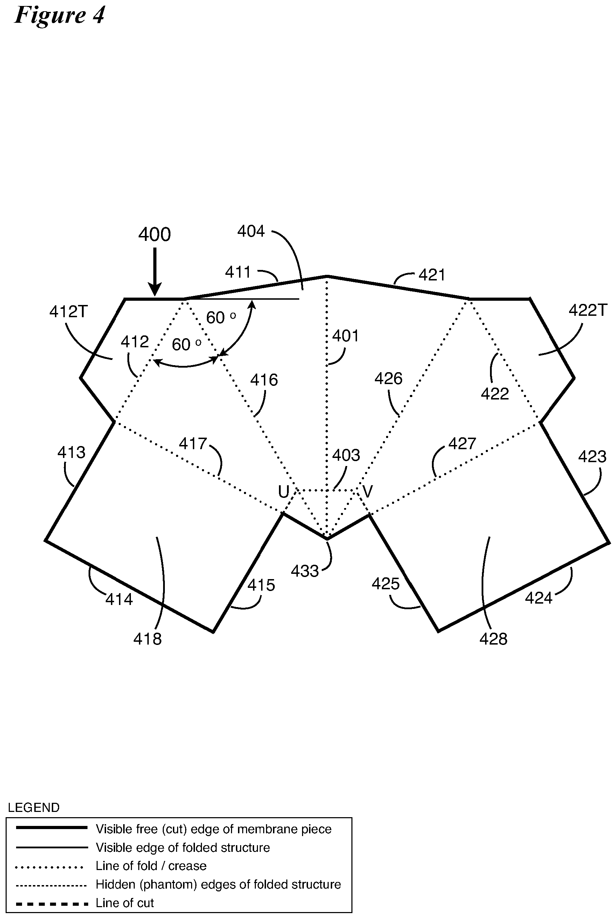

Referring now to Fig. 4, and in accordance with at least one embodiment, a plan view of pattern 400 is shown for a flat sheet membrane similar to pattern 300, except that the extension "tab" sections 412T and 422T are distal extensions of the cusp wall sections only. This limitation reduces the double layer of membrane extension at the distal end of the completely folded integrated cusp and leaflet structure to a single layer, thereby reducing the mass of membrane in the heart valve which might otherwise disadvantageously limit the efficiency of collapsing and compressing the valve for use in the percutaneous/transcatheter delivery application.

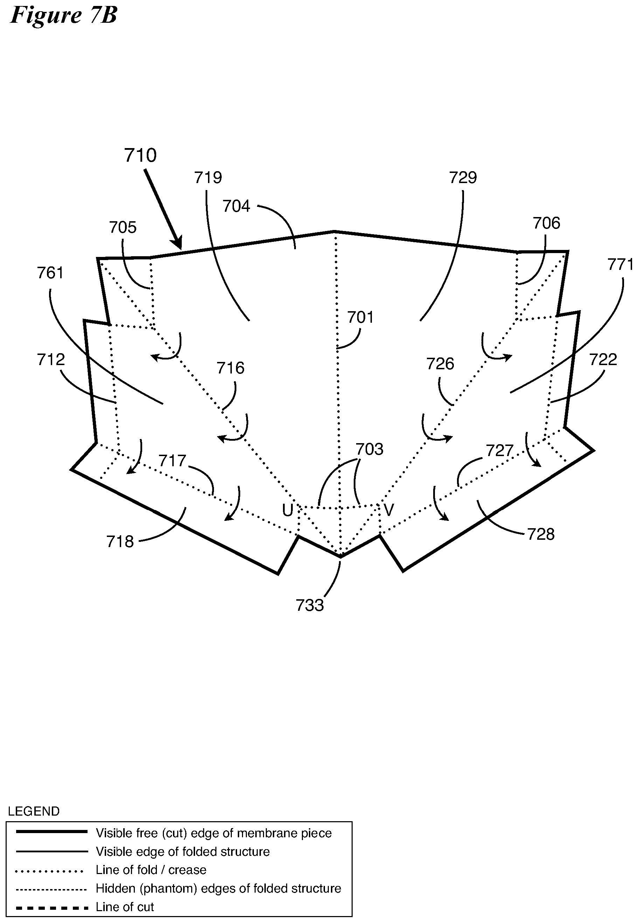

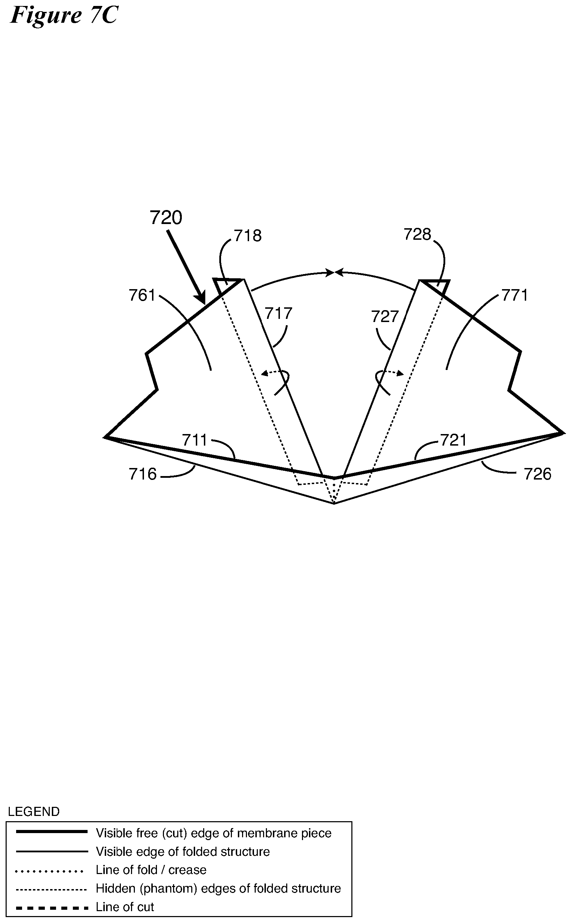

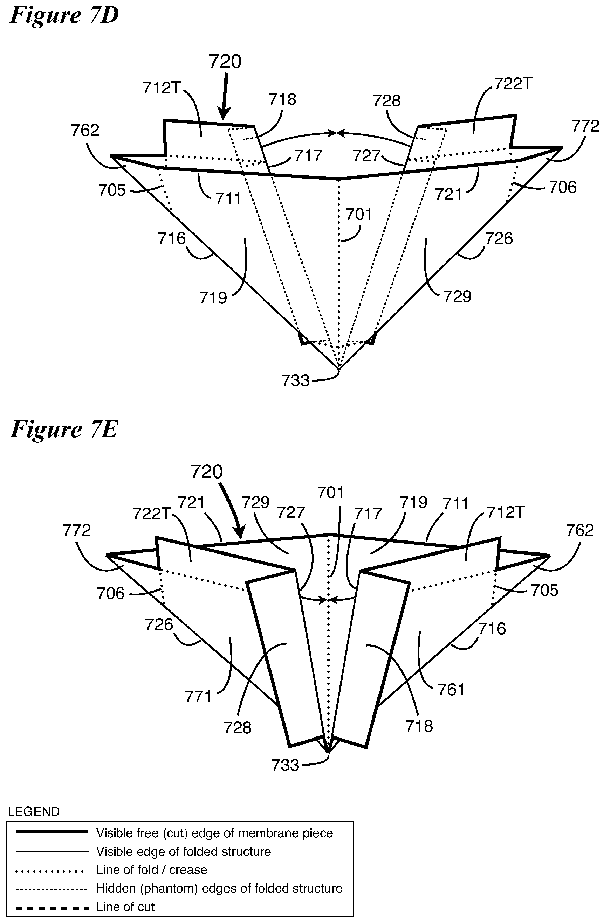

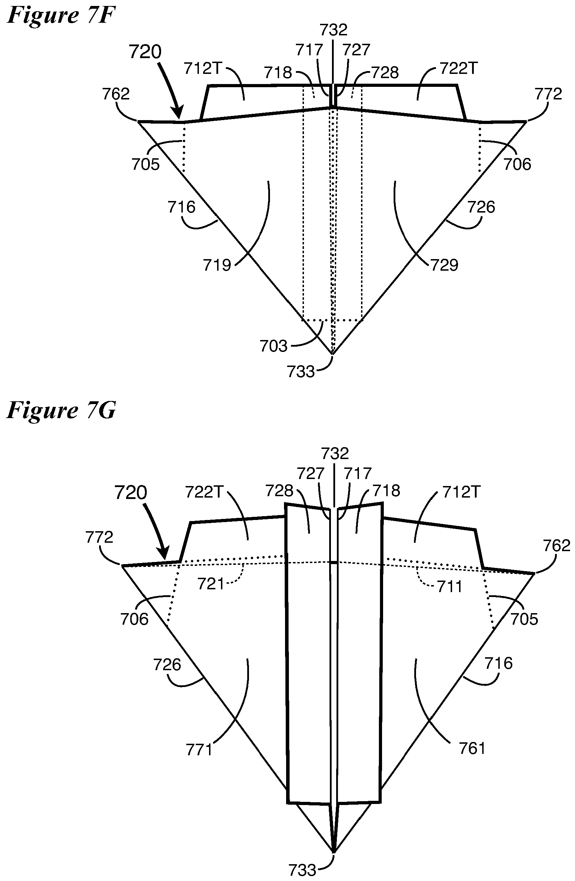

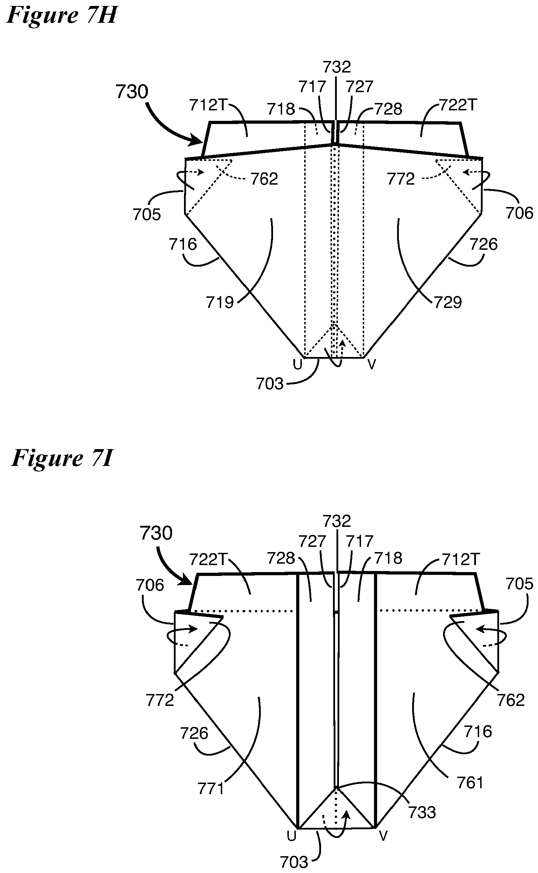

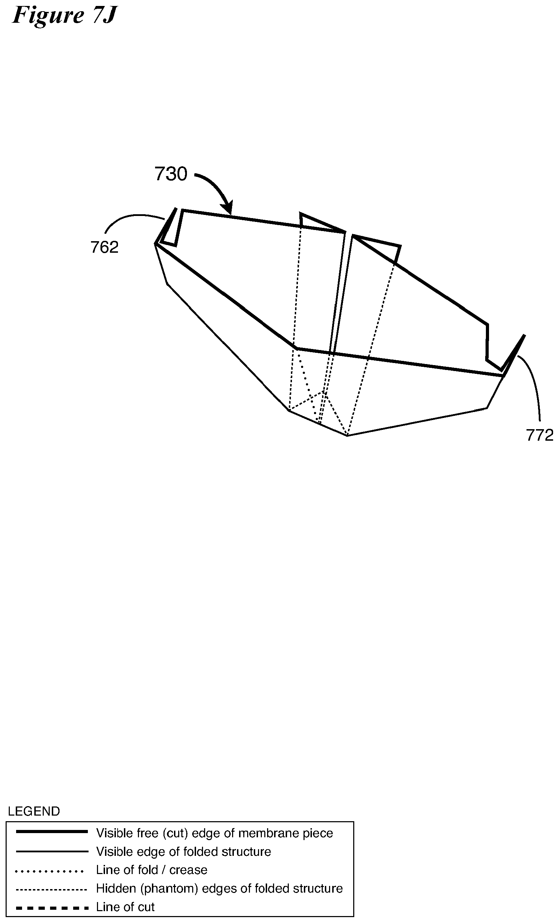

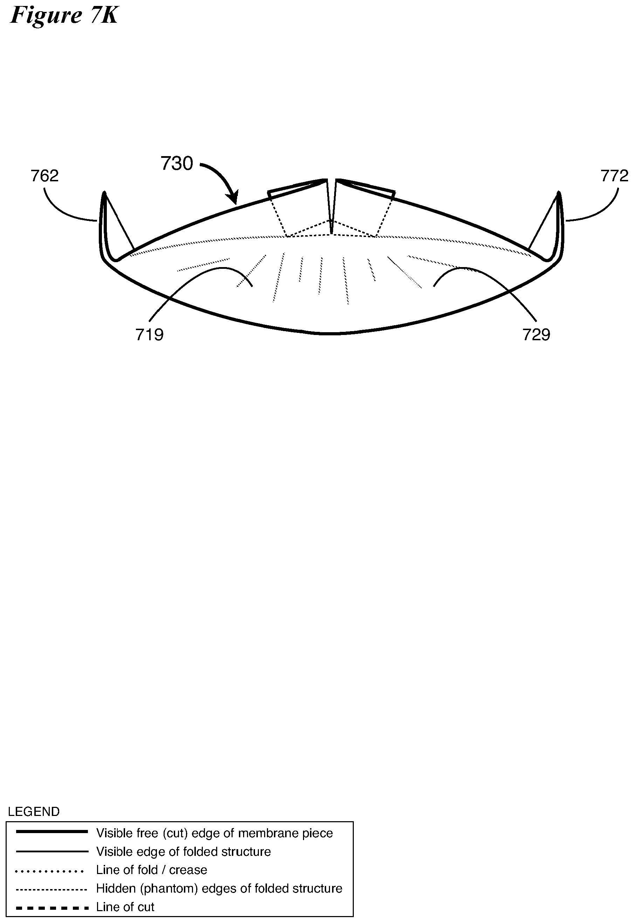

In addition, at the lower (proximal) apex 433 of the cusp cone pattern the lower (proximal) extent of the cuff wall sections 418 and 428 is limited so as to "expose" the apex of the cone in the pattern. This feature allows, on the completely folded integrated cusp and leaflet structure, the transverse, radially outward folding of the tip of the cone-shaped cusp at line 403 between points U and V. (See figures 7.) The folding of the apex reduces the overall axial length of the cusp and leaflet structure, allowing for increased cusp/sinus volume for a given valve diameter and frame length.

The template 400 contains folds 401, 416, 426, 417 and 427 that correspond to folds 101, 116, 126, 117 and 127, respectively, and are disposed in like manner in folding execution, as are the edge segments similarly enumerated. Similar to templates 200 and 300 described above, template 400 dimensions may be altered to suit the particular clinical application of the valve. The example is shown for a cusp cone wall disposed at about a 60 degree angle to the horizontal (short axis) of the generally cylindrical valve geometry.

### Folded Valve Pattern Variation No. 5

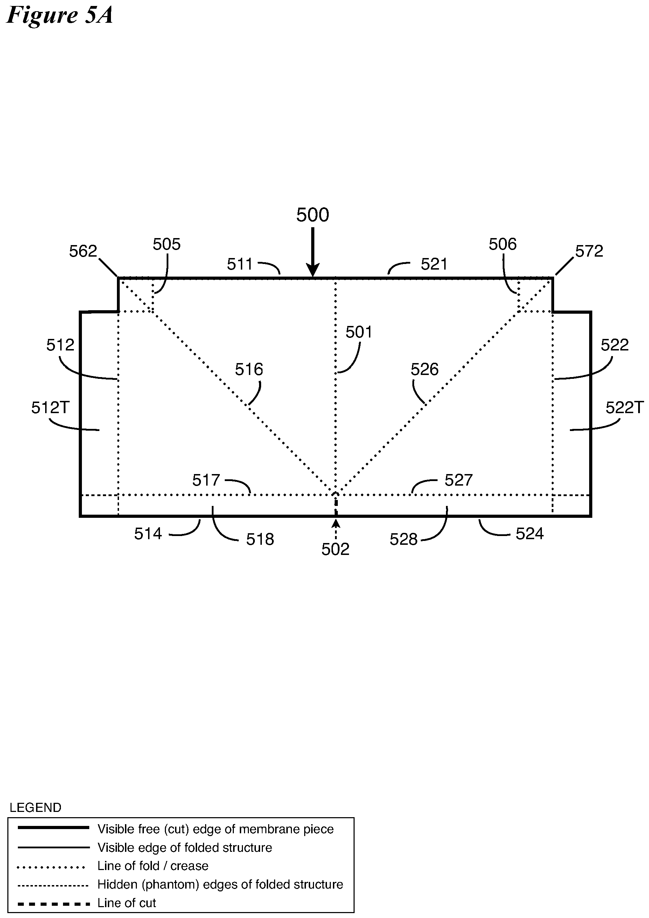

Referring now to Figs. 5A-5F, yet another embodiment of a template pattern is illustrated. Referring specifically now to Fig. 5A, a plan view of template 500 is shown for a flat sheet membrane. The template 500 contains folds 501, 516, 526, 517 and 527 that correspond to folds 101, 116, 126, 117 and 127, respectively, and are disposed in like manner in folding execution, as are the edge segments similarly enumerated.

Template 500 illustrates a flat sheet membrane that is basically rectangular and is similar to the upper (distal) portion of template 100 of Figs. 1A-1G, except that (a) the distal extension areas 512T and 522T are added at the left and right margins of the template 500, and (b) the lower quadrants forming the cuff wall sections of the template 100 are truncated in template 500 to narrow cuff wall sections 518 and 528, the extent of which is defined by the length of cut 502. These limited interior cuff sections are still used for frame attachment along the central seam 532 of the cusp and leaflet cone, and the distal extension sections 512T and 522T are still used for attachment of the outer cusp wall to the distal edge of the frame.

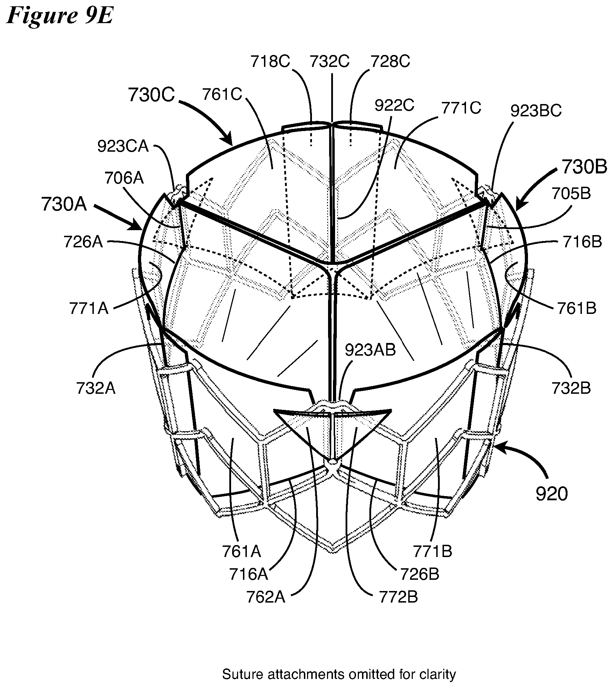

Referring still to Figs. 5A-5F, corner folds 505 and 506 are now described. For template 500, after folds 516 and 526 are executed by complete folding, segments 512 and 522 are apposed and aligned to segments 511 and 521, respectively, and overlapping layers (mobile leaflet layer and cusp wall layer) form triangular corner sections at 562 and 572. Radially outward folding of these corner sections at 505 and 506 define the axial extent of the leaflet commissures such that joining the corner sections of adjacent leaflet structures along corner folds 505 and 506 causes the leaflet apposition to be at least the length of 505 in axial extent at the radial margin of the leaflet. (See Fig. 9E that illustrates an embodiment of a valve comprising a frame 920 with a plurality of integral cusp and leaflet structures 730 attached to the frame, wherein the structures 730 include corner sections 762 and 772 corresponding to the corner sections 562 and 572 of template 500.) Additionally, these double-layer triangular corner sections 562 and 572 are used for attachment of the commissures to the frame. The stent frame may optionally contain a slot at this point of attachment through which this triangular "tab" section may be inserted and attached on the abluminal surface of the frame. (Again, see Fig. 9E.)

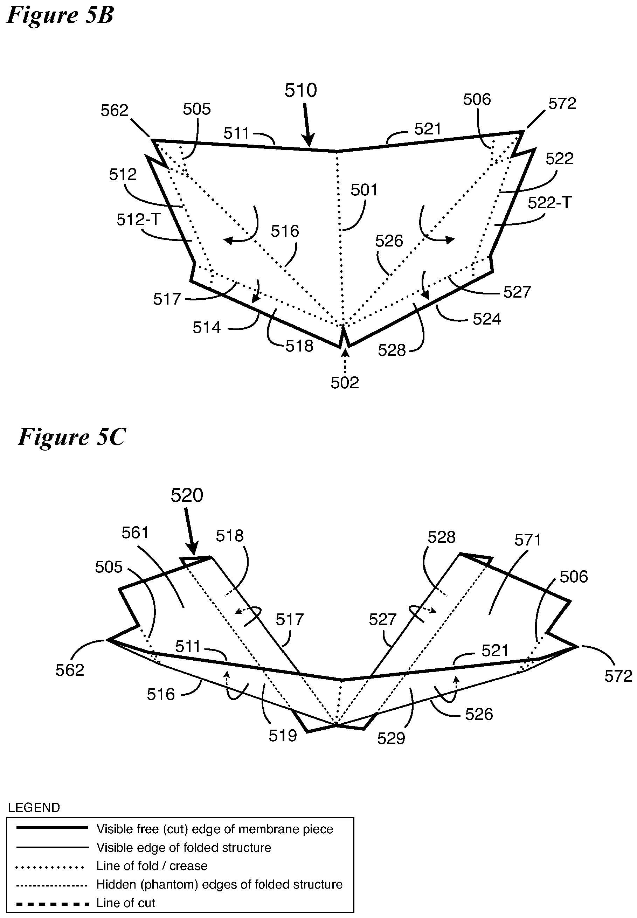

With specific reference now to Fig. 5B, a perspective view of the inner aspect (that is, a view directed radially outward) of an initially folded structure 510 folded according to template 500 is shown. The central folding along 501 is initiated after cut 502 is executed as shown. Foldings along 501, 516, and 526 are depicted as initiated radially inward (out of the page) and foldings along 517 and 527 are depicted as initiated radially outward (into the page).

Figure 5C shows a steeply oblique perspective view of the folded integrated cusp and leaflet 520 at an intermediate stage of completion of the foldings. The view is directed from the central axis outward and obliquely downward into the cusp space showing the formation of the outer wall of the structure, that is, the cusp wall layer of the subject cusp. Folding along 517 and 527 acts to position the extension sections 518 and 528 outward of the cusp wall sections 561 and 571, respectively. Completion of folding then will position folds 517 and 527 in an axially aligned orientation in apposition to each other along their length. Folding along 516 and 526 acts to position the cusp wall sections 561 and 571 outward of the mobile leaflet sections 519 and 529, respectively. Completion of folding, which radially collapses the folded flattened structure, positions the cusp wall sections 561 and 571 in apposition to the mobile leaflet sections 519 and 529, respectively. In the final folded configuration the structure embodies the integrated cusp and leaflet in the open operating position.

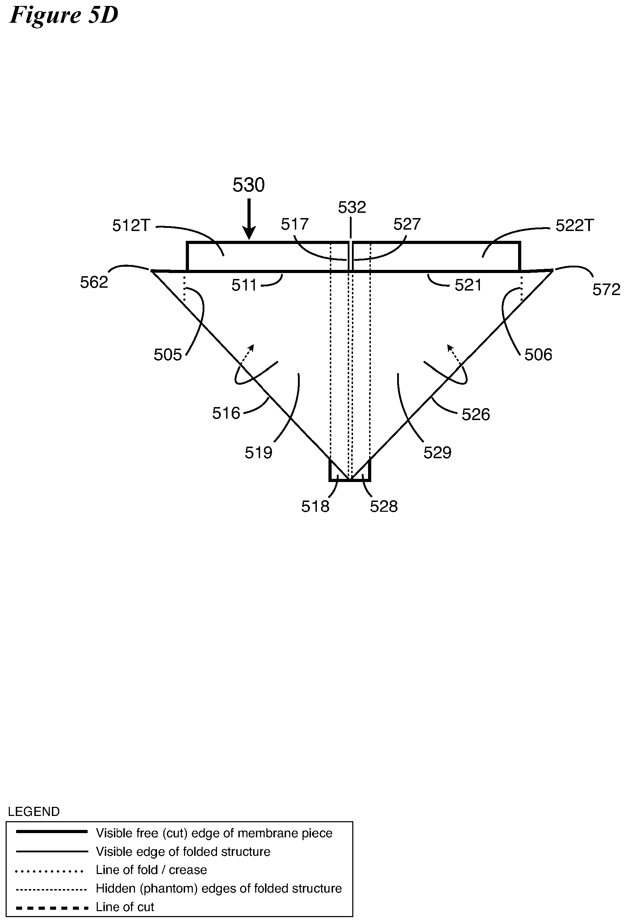

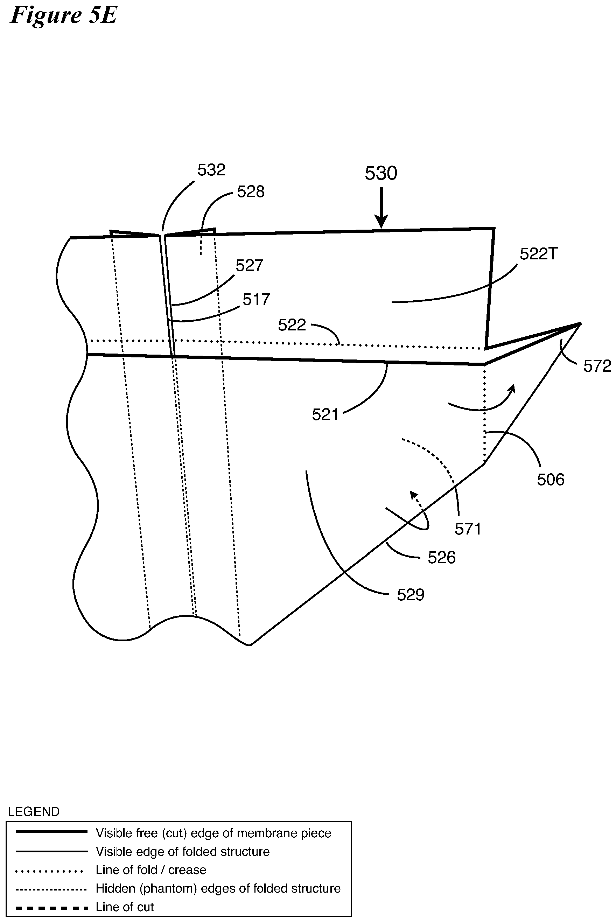

In addition, completed folding at 516 and 526 also forms triangular two-layer sections, 562 and 572, respectively, that are designated as "commissure tabs". These commissure tabs are bounded by the corner folds 505 and 506, folds 516 and 526, and the free edges 511 and 521 of the mobile leaflet sections 519 and 529, respectively. With further reference to Figs. 5D and 5E, these commissure tabs will be folded at 505 and 506 so as to position both layers of the tabs outward of the cusp wall sections 561 and 571, respectively, with the folds 505 and 506 oriented parallel to the central axis of the valve. With regard to a multi-leaflet valve, when the cusp and leaflet structure is mounted within the frame, this folded commissure tab is aligned along fold 505 in apposition to fold 506 of an adjacent complementary commissure tab of an adjacent integrated cusp and leaflet structure. Thus mounted, the commissure tabs join the mobile leaflet layers and the cusp wall layers of adjacent folded cusp and leaflet structures along a line coincident to both 505 and 506 that forms a common seam for attachment, such as by suturing of the commissure tabs to each other and to the frame forming the circumferential margin of the membrane portion of the folded cusp and leaflet structure.

Fig. 5D shows a plan view of the inner (luminal) aspect of the folded integrated cusp and leaflet structure 530 of template pattern 500. Structure 530 is depicted in a completed state of folding, excepting that the commissure tabs 562 and 572 are not yet folded outward along fold lines 505 and 506, respectively. The radially flattened form shown gives the general configuration and orientation of the membrane segments and sections for the open operating position of the valve cusp and leaflet.

Still referring to Fig. 5D, at the uppermost (distal) portion of the cusp wall layer, the extension tabs 512T and 522T are projected above (or distal to) the lines 512 and 522 (shown in figures 5A and 5B), respectively, that lie in apposition and alignment to the free edges 511 and 521, respectively, of the mobile leaflet layer. A portion or all of these tabs 512T and 522T may be optionally folded outward along 512 and 522, respectively, around the distal edge of the frame to lie upon the outer (abluminal) surface of the frame where they may be attached to both the frame and to the cusp wall sections (where the cusp wall sections are apposed to the inner surface of the frame) through the interstices of the frame. This optional configuration provides for increased strength of attachment for bearing downward (proximally directed) operational loads associated with the valve closing.

Completing the folding associated with template pattern 500 places folds 517 and 527 into axial alignment. Once in axial alignment, apposing folds 517 and 527 are joined along their axial length to form the seam 532 that closes the generally conical cusp structure with the extension sections 518 and 528 situated outward of the cusp wall sections 561 and 571, respectively. The cusp wall sections 561 and 571 are thus disposed outward of the mobile leaflet sections 519 and 529, respectively, with the cusp wall sections axially and circumferentially apposed to the inner surfaces of the generally cylindrical frame. Advantageously, for each valve cusp and leaflet to be mounted within, the frame may contain an element or elements that are axially oriented and span a significant portion of the axial length of the frame, so as to align with the seam 532 for attachment, such as by suturing to the frame.

Referring now to Fig. 5E, a partial detail perspective view is shown of the commissure tab 572 configuration of the completely folded integrated cusp and leaflet structure 530, indicating radially outward folding of the commissure tab 572 along fold line 506.

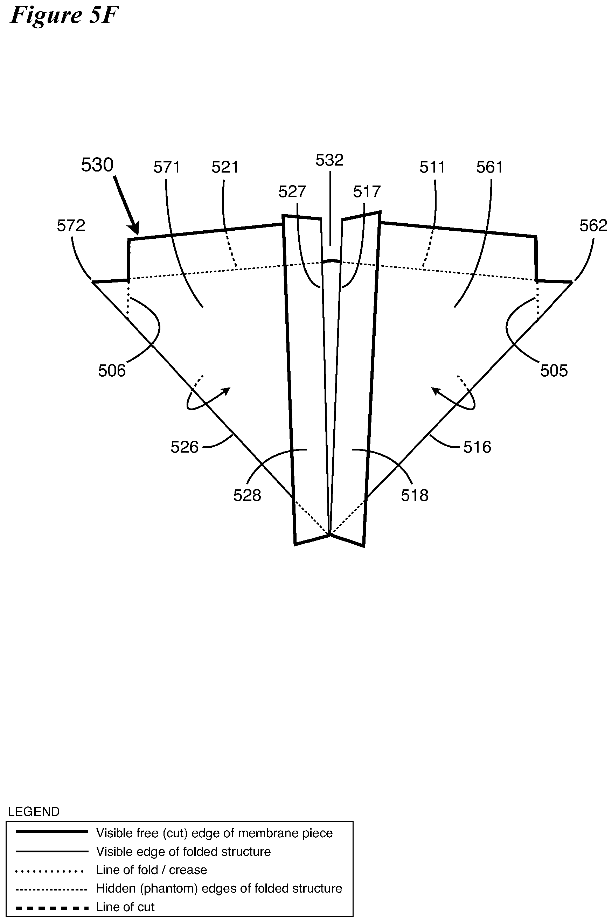

With reference now to Fig. 5F, a perspective view is shown of the outer (abluminal) aspect of the completely folded cusp and leaflet structure 530 (except that the triangular commissure tabs are not yet folded) of template 500 in substantially flattened form. This view is complementary to Fig. 5D that shows the inner aspect of the same structure 530. The central seam 532 is seen on the outer face of the cusp wall sections 561 and 571 and is depicted for purposes of illustration as partly separated with the extension sections 518 and 528 incompletely flattened and folds 517 and 527 in close, but not in the complete apposition and alignment that will form the final seam line 532 for attachment to the axially oriented frame members. The slight separation depicted between folds 517 and 527 exposes the centerpoint of the mobile leaflet free edge where the mobile leaflet free edge segments 511 and 521 meet as depicted behind the cusp wall sections 561 and 571, respectively, in this view.

### Folded Valve Pattern Variation No. 6

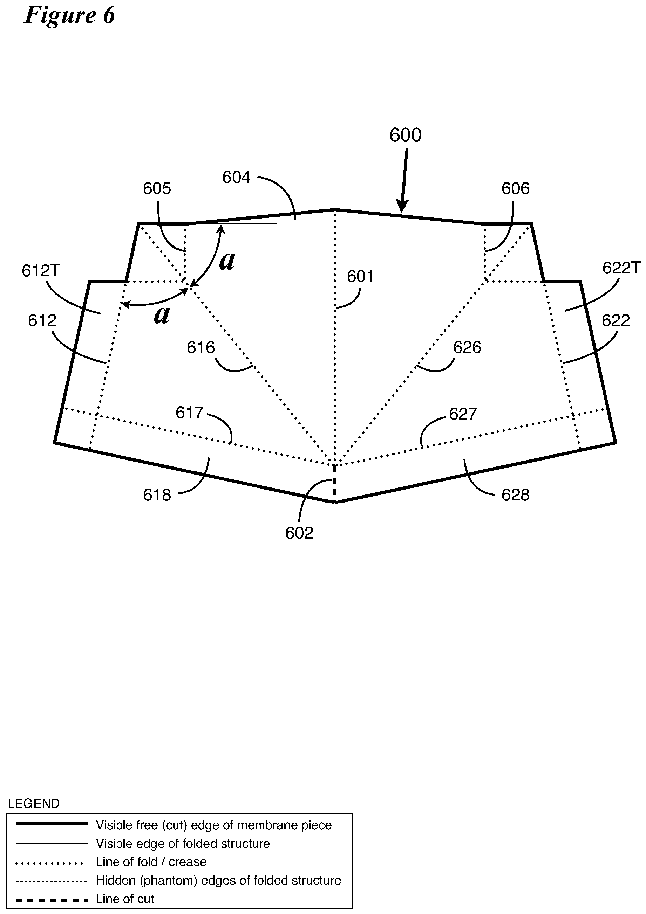

In accordance with at least one embodiment, Fig. 6 shows a plan view of another template 600 that is similar to template 500 except that the cusp cone wall angle ***a*** exceeds the 45 degrees of the generally rectangular template 500, and that the mobile leaflet sections are extended by a polygonal or curved extension section 604 of the free edge.

The change in cusp cone wall angle ***a*** also results in changes in the angle relating the lower (proximal) margins of the template and fold lines 617 and 627 to the center line of the template in order that when folding is completely executed, the fold lines 617 and 627 and the seam between them will be parallel to the central axis of the assembled valve. Likewise, the further geometry of the cusp cone wall angle will result in fold lines (optional) 613 and 623 and the long axes of extension tabs 612T and 622T being parallel to the transverse axis of the assembled valve.

The template 600 contains folds 601, 616, 626, 617, 627, optional folds 612 and 622, corner folds 605 and 606, and cut line 602 that correspond to folds 501, 516, 526, 517, 527, optional folds 512 and 522, corner folds 505 and 506, and cut line 502, respectively, of template pattern 500 and are disposed in like manner in folding execution, as are the template sections and edge segments similarly enumerated.

### Folded Valve Pattern Variation No. 7

Referring now to Figs. 7A-7F, still yet another embodiment of a template pattern is illustrated. Referring specifically now to Fig. 7A, a plan view of another template 700 is shown that is similar to template 600, but with a section of the lower (proximal) midline portion of the template cut away so as to expose the apex 733 of the triangular sections that, when folded, will form the apex of the cone-shaped cusp. Effectively, the midline portions of the extension sections 718 and 728 are removed in relation to template 600 to an extent determined by the desired length of the line segment U-V, which in turn determines the extent to which the apex of the cone-shaped cusp may be truncated by folding at U-V.

After the cusp and leaflet cone is formed by folding, the apex is folded radially outward at line U-V (703) to truncate the cone to reduce the overall length of the cusp and leaflet structure, allowing for increased cusp/sinus volume for a given valve diameter and frame length.

The template 700 contains folds 701, 716, 726, 717, 727, optional folds 712 and 722, and corner folds 705 and 706, that correspond to folds 601, 616, 626, 617, 627, optional folds 612 and 622, and corner folds 605 and 606, respectively, of template 600 and are disposed in like manner in folding execution, as are the template sections and edge segments similarly enumerated.

Fig. 7B shows a perspective view of the inner (luminal) aspect of the initially folded cusp and leaflet structure 710 of template 700 after initiation of the principal folds 716, 726, 717, 727 and 701. Inward folding along 701 assists in aligning the left and right sections of the structure, but is not necessary to the formation of the integrated cusp and leaflet folded structure or to the operation of the valve. The disposition of the folds that converge at the apex 733 of the cusp can be appreciated as later forming an overlapping two-layer triangular apex as the cusp wall sections 761 and 771 are folded outward along lines 716 and 726, respectively, so as to position the cusp wall sections 761 and 771 outward of, and in apposition to, the mobile leaflet sections 719 and 729, respectively.

Fig. 7C shows a steeply oblique perspective view of the folded integrated cusp and leaflet 720 at an intermediate stage of completion of the foldings. The view is directed from the central axis outward and obliquely downward into the cusp space showing the formation of the outer wall of the structure. Folding along 717 and 727 acts to position the extension sections 718 and 728 outward of the cusp wall sections 761 and 771, respectively. Completion of folding then will position folds 717 and 727 in an axially aligned orientation in apposition to each other along their length. Folding along 716 and 726 acts to position the cusp wall sections 761 and 771 outward of the mobile leaflet sections 719 and 729, respectively. Completion of folding, which radially collapses the folded flattened structure, positions the cusp wall sections 761 and 771 in apposition to the mobile leaflet sections 719 and 729, respectively. In the final folded configuration, the structure embodies the integrated cusp and leaflet in the open operating position.

With reference to Fig. 7D, completed folding at 716 and 726 also forms triangular two-layer sections, 762 and 772, respectively, that are designated as "commissure tabs." These commissure tabs are bounded by the corner folds 705 and 706, folds 716 and 726, and the free edges 711 and 721 of the mobile leaflet sections 719 and 729, respectively. With further reference to Figs. 7D and 7E, these commissure tabs will be folded at 705 and 706 so as to position both layers of the tabs outward of the cusp wall sections 761 and 771, respectively, with the folds 705 and 706 oriented parallel to the central axis of the valve. When the integrated cusp and leaflet structure is mounted within the frame, this folded commissure tab is aligned along fold 705 in apposition to fold 706 of an adjacent complementary commissure tab of an adjacent integrated cusp and leaflet structure of a multi-leaflet valve. Thus mounted, the commissure tabs join the mobile leaflet layers and the cusp wall layers of adjacent folded cusp and leaflet structures along a line coincident to both 705 and 706 that forms a common seam for attachment, such as by suturing of the commissure tabs to each other and to the frame forming the circumferential margin of the membrane portion of the folded cusp and leaflet strucutre.

Fig. 7D shows a perspective view of the inner (luminal) aspect of the partially folded integrated cusp and valve structure 720 of template 700. Integrated cusp and leaflet structure 720 is depicted in nearly completed state of folding, except that the commissure tabs 762 and 772, as well as the cusp apex 733 are not yet folded outward along fold lines 705, 706 and 703, respectively, and that the axial seam 732 is not yet formed by the apposition of the folds 717 and 727.

At the uppermost (distal) portion of the cusp wall layer, the extension tabs 712T and 722T are projected above (or distal to) the lines 712 and 722 (shown in Figs. 7A and 7B). All or a portion of these tabs 712T and 722T may be optionally folded outward along 712 and 722, respectively, around the distal edge of the frame to lie upon the outer (abluminal) surface of the frame where they may be attached to both the frame and to the cusp wall sections (apposed to the inner surface of the frame) through the interstices of the frame. This optional configuration provides for increased strength of attachment for bearing downward (proximally directed) operational loads associated with the valve closing.

Completing the folding associated with template pattern 700 places folds 717 and 727 into axial alignment. Once in axial alignment, apposing folds 717 and 727 are joined along their axial length to form the seam 732 that closes the generally conical cusp structure with the extension sections 718 and 728 situated outward of the cusp wall sections 761 and 771, respectively. The cusp wall sections 761 and 771 then are disposed outward of the mobile leaflet sections 719 and 729, respectively, with the cusp wall sections axially and circumferentially apposed to the inner surfaces of the generally cylindrical frame. Advantageously, for each integrated cusp and folded leaflet structure to be mounted within, the frame may contain an element or elements that are axially oriented and span a significant portion of the axial length of the frame, so as to align with the seam 732 for attachment as by suturing to the frame.

Fig. 7E shows a shallow oblique top perspective view of the outer (abluminal) aspect of the partially folded cusp and leaflet structure 720 of template 700 (except that the triangular commissure tabs 762 and 772 and apex 733 are not yet folded and that the axial seam 732 is not yet joined). This view is complementary to Fig. 7D that shows the inner aspect of the same structure 720. The central seam 732 will be formed on the outer face of the cusp wall sections 761 and 771 as folds 717 and 727 are brought together into apposition along the midline, with the extension sections 718 and 728 thus also aligned. The outward (abluminal) face of the mobile leaflet sections 719 and 729 are shown between the yet separated folds 717 and 727 before closure of the generally conical cusp along the outer seam 732.

Fig. 7F shows a plan view of the inner (luminal) aspect of the folded integrated cusp and leaflet structure 720 of template pattern 700. Structure 720 is depicted in a completed state of folding, excepting that the commissure tabs 762 and 772 are not yet folded outward along fold lines 705 and 706, respectively. In addition, the apex 733 is not folded outward. The radially flattened form shown gives the general configuration and orientation of the membrane line segments and areal sections for the open operating position of the valve cusp and leaflet.

At the uppermost (distal) portion of the cusp wall layer, the extension tabs 712T and 722T are projected above (distal to) the lines 712 and 722 (shown in Figs. 7A, 7B and 7G), respectively, below (proximal to) which the cusp wall sections 761 and 771 lie in radial apposition to the mobile leaflet sections 719 and 729, respectively, of the mobile leaflet layer. These tabs 712T and 722T may be optionally folded outward along 712 and 722, respectively, around the distal edge of the frame to lie upon the outer (abluminal) surface of the frame where they may be attached to both the frame and to the cusp wall sections (apposed to the inner surface of the frame) through the interstices of the frame. This optional configuration provides for increased strength of attachment for bearing downward (proximally directed) loads of valve closing.

Folding of the template positions folds 717 and 727 into axial alignment, joined along their axial length to form the seam that closes the generally conical cusp structure with the extension sections 718 and 728 reflected outward of the cusp wall sections 761 and 771, respectively. The cusp wall sections 761 and 771 then are disposed outward of the mobile leaflet sections 719 and 729, respectively, with the cusp wall sections 761 and 771 axially and circumferentially apposed to the inner surfaces of the generally cylindrical frame. Advantageously, for each valve cusp and leaflet folded structure to be mounted within, the frame may contain an element or elements that are axially oriented and span a significant portion of the axial length of the frame, so as to align with the seam 732 for attachment as by suturing to the frame.

Fig. 7G shows a perspective view of the outer (abluminal) aspect of the completely folded cusp and leaflet structure 720 (except that the triangular commissure tabs 762 and 772 and apex 733 are not yet folded) of template 700, in nearly flattened form. This view is complementary to Fig. 7F that shows the inner aspect of the same structure 720. The central seam 732 is seen on the outer face of the cusp wall sections 761 and 771 and is depicted for purposes of illustration as minimally separated with the extension sections 718 and 728 incompletely flattened and folds 717 and 727 in effectively complete apposition and alignment that forms the final seam line 732 for attachment to the axially oriented frame members. The slight separation depicted between folds 717 and 727 exposes the centerpoint between the mobile leaflet free edge segments 711 and 721 depicted behind the cusp wall sections 761 and 771, respectively, in this view.

Fig. 7H shows a plan view of the inner aspect of the completely folded integrated cusp and leaflet structure 730 of template 700. This view is substantially that of Fig. 7F except that the triangular commissure tabs 762 and 772 are folded radially outward of the cusp wall sections 761 and 771 along corner folds 705 and 706, respectively. Additionally, the apex (most proximal) portion of the cone-shaped cusp is folded radially outward along the fold line 703 (between points U and V) to the position radially outward of the joined extension sections 718 and 728 such that the apex point 733 then lies upon the seam line 732.

Fig. 7I shows a plan view of the radially outer aspect of the completely folded integrated cusp and leaflet structure 730 of template 700. The outwardly folded position of the triangular commissure tabs 762 and 772 can be seen so that they lie in apposition to the outer surface of the cusp wall sections 761 and 771, respectively. While they may attached in this position to the underlying cusp wall layer and to the frame, alternatively, the commissure tabs 762 and 772 may be positioned to point radially outward (out of the page in this view) to pass through a slot or space in the frame to be secured and attached to the outer (abluminal) surface of the frame.

Additionally, the apex (most proximal) portion of the cone-shaped cusp is folded radially outward along the fold line 703 (between points U and V) to the position radially outward of the joined extension sections 718 and 728 such that the apex point 733 then lies upon the seam line 732.

The apex portion of the cone-shaped cusp thus configured is to be attached in this position as by suturing and may be similarly attached into this position in the act of attaching or suturing this portion of the folded cusp and leaflet structure to the frame.

Fig. 7J shows an oblique top perspective view of the completely folded and formed cusp and leaflet structure 730 with the view directed radially outward and downward (proximal). The cusp and leaflet structure is shown with the free edge of the mobile leaflet layer in the inward central position corresponding to the substantially closed operating position of the valve leaflet.

The commissure tabs 762 and 772 are depicted in radially aligned positions directed outward as would be required for passing them through slots or spaces in a suitably designed frame.

Fig. 7K shows a top perspective view of the single-piece completely folded and formed cusp and leaflet structure 730 with the view directed downward (proximal) into the cusp space. The cusp and leaflet structure is shown with the free edge of the mobile leaflet layer sections 719 and 729 in the intermediate inward position corresponding to the partially closed operating position of the valve leaflet.

The membrane structure is depicted with the free edges in a relaxed state corresponding to the typical behavior of tissue membranes when hydrated as when implanted in the body.

The commissure tabs 762 and 772 are depicted in radially aligned positions directed outward as would be required for passing them through slots or spaces in a suitably designed frame.

### Metal Lattice Frame

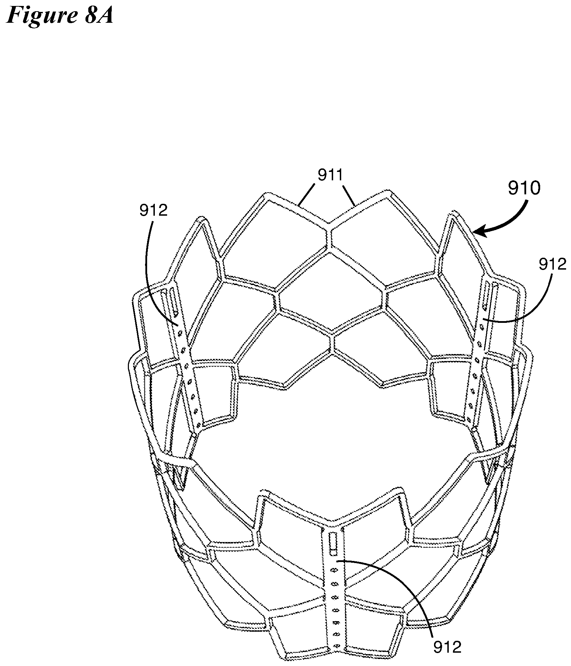

Fig. 8A is an oblique top perspective view of a metal lattice frame 910 for mounting three of the single-piece folded integrated cusp and leaflet structures of the ensuing description in order to form a three-leaflet valve. The frame comprises a plurality of strut members 911 and three axially oriented mounting bars 912 each with holes and/or slots for passing suture and/or portions of the folded membrane structure. Each mounting bar 912 is to align with and attach to the axial outer seam of one single-piece completely folded and formed cusp and leaflet structure 730. The diameter D of the open frame, e.g., 19 - 35 mm naturally defines the deployed and operating diameter of the valve assembly after implantation in the body. The strut members 911 are of specific length and orientation to permit radial collapse and compression of the frame to a small diameter, e.g., 3-7 mm. The mounting bars 912 are near to equally spaced around the circumferential course of the frame and the length L of the arc from the center of the mounting bar 912 to the center of the closest mounting bar 912 is approximately equal to (pi x D)/3. Thus defined, L also defines the transverse circumferential distance between folds 705 and 706 , approximating the circumferential extent of the portions of the joined cusp wall sections 761 and 771 extending between 705 and 706 of the folded cusp and leaflet structure of appropriate size when mounted within the frame 910.

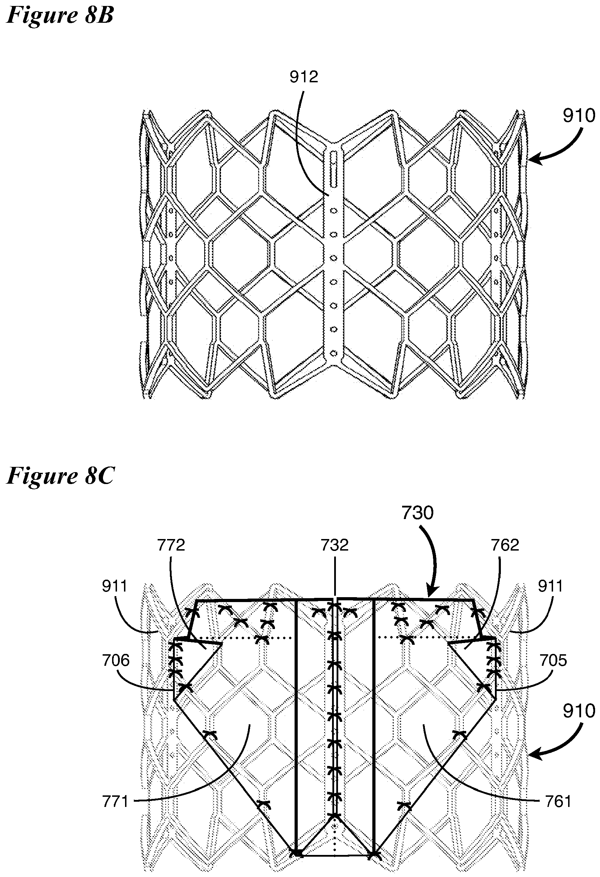

Fig. 8B shows a side perspective view of the frame 910 with the view centered on the axial mounting bar 912. The axial bars are shown with holes and/or slots for passing suture and/or portions of the folded membrane structure to enable secure mounting of the folded cusp and leaflet structure within the frame.

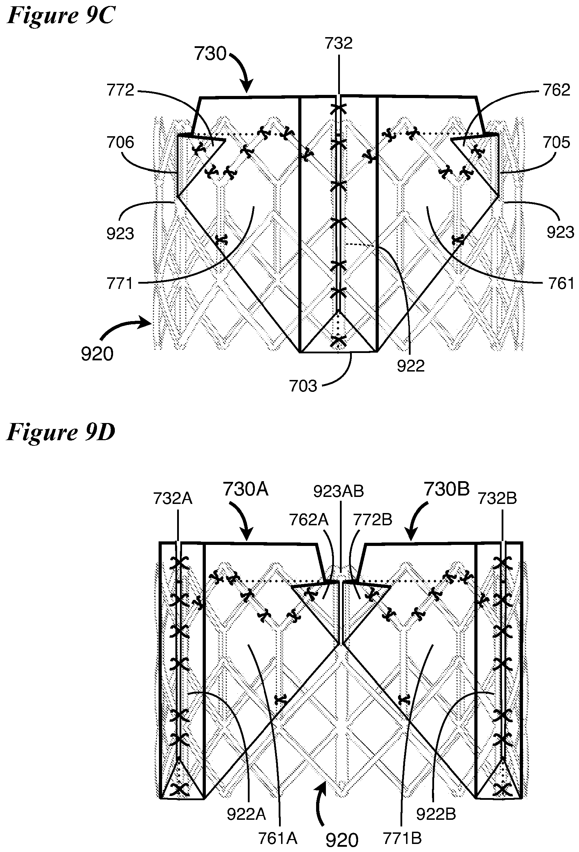

Fig. 8C shows a side view of the frame of Fig. 8B with a superimposed plan view of the radially outer aspect of the completely folded integrated cusp and leaflet structure as depicted in Fig. 7I. The cusp wall seam 732 is aligned upon the inner surface of the mounting bar of the frame and attached by sutures in this example. (Example suture locations are shown in Figs. 8C, 9C and 9D shown with an "x"; however, it is to be understood that the locations shown are exemplary and not limiting.) As those skilled in the art will appreciate, means other than sutures for attaching folded integrated cusp and leaflet structure to the frame can be used.

The commissure tabs 762 and 772 are folded flat against the outer surface of the cusp wall layer along corner folds 705 and 706 for mounting entirely within the frame 910. Each fold 705 then forms an axially oriented seam along its length with the complementary fold 706 of the adjacent folded cusp and leaflet structure 730. (Adjacent complementary commissure tabs omitted for clarity.) Said seam is closed and attached by suture, for example, while also attaching to the radially overlying strut member 911 of the frame 910, and thereby affixes the distal margins of the cusp wall sections 761 and 771 and the mobile leaflet sections (obverse of this view) to the frame 910. The other suture points depicted attach only the cusp wall layer 761+771 to the overlying frame strut members 911. At no point within the interior operating volume of the valve is the mobile leaflet layer 719+729 penetrated by suture. This uninterrupted continuity of the operating leaflet material afforded by the folded design of the integrated cusp and leaflet structure endows the valve and its leaflets with strength, durability and resistance to stress damage at suture holes.

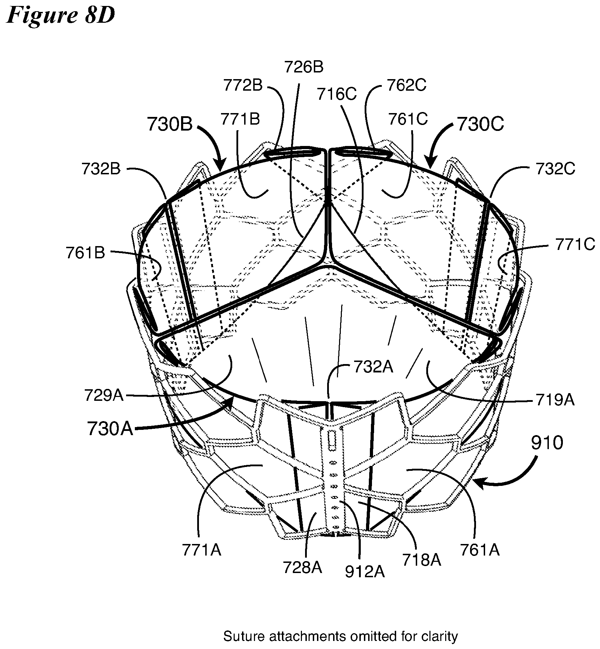

Fig. 8D shows an oblique axial (top/distal) perspective view of the assembled three-leaflet valve comprising the frame 910 and three identical folded integrated cusp and leaflet structures 730A, 730B and 730C attached within the frame with the view centered on an axial mounting bar 912A. The suture attachments are omitted for clarity. The cusp and leaflet structure 730A nearest in view is seen within the frame 910, with the outer aspect of the seam 732A, cuff wall extension sections 718A and 728A, and cusp wall sections 761A and 771A viewed through the interspaces of the frame 910. The seam 732A is aligned to the overlying axial mounting bar 912A to which it is attached along its length. The inner (luminal) aspect of the seams 732B and 732C and the cusp wall sections 761B, 771B, 761C and 771C of the other two folded cusp and leaflet structures 730B and 730C, respectively are seen on the far side of the view. The adjoined folded edges of the membrane portions of the commissure tabs 772B and 762C are shown in the far view in position opposite to the axial mounting bar 912A in the near view. The radially outward surface of the mobile leaflet sections 719A and 729A of the folded cusp and leaflet structure 730A is shown in the near view. The distal free edges of all three mobile leaflets are shown in the centrally apposed (coapted) position corresponding to the closed operating position of the valve. Fig. 8D also shows in that aspect interior to the cusps, folds 726B of cusp and leaflet structure 730B and 716C of cusp and leaflet structure 730C as they form the lower (proximal) boundary of the valve cusps.

### Slotted Lattice Frame

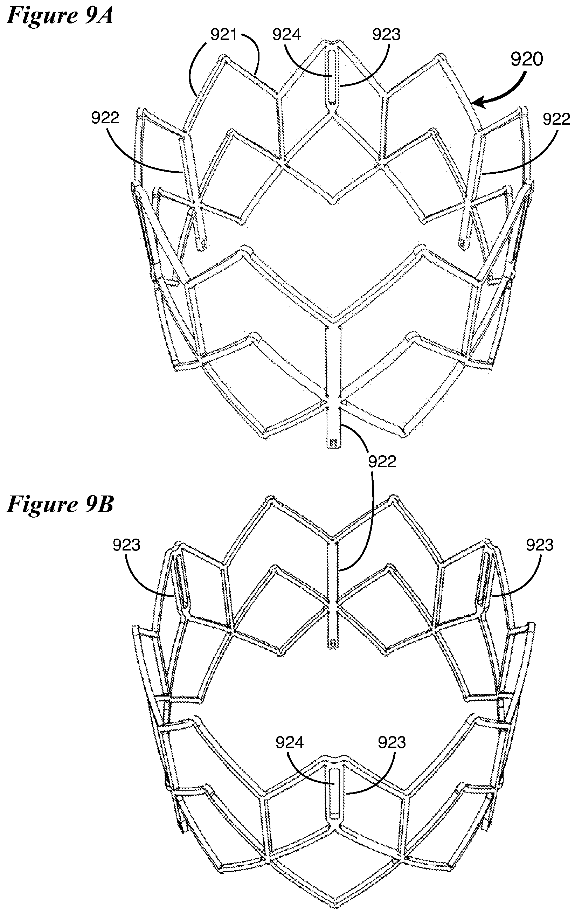

Fig. 9A shows an oblique axial (top/distal) perspective view of a frame 920 of a design to receive the commissure tabs 762 and 772 through slots 924 in slotted members 923 in order that the tabs are secured and attached to the outer (abluminal) aspect of the frame. This approach to mounting and attaching the commissure tabs enables the loading forces on the leaflet commissures during valve operation to be advantageously distributed upon the frame slotted members 923 along their length rather than upon suture that directly tethers the leaflets, thus greatly reducing the risk of tearing of the material at points of suture penetration. The frame further comprises axial mounting bars 922 for mounting the central seams 732 joining the cusp wall sections 761 and 771 along folds 717 and 727. The frame further comprises a plurality of strut members 921 that otherwise form the metal lattice of the frame.

Each mounting bar 922 is to align with and attach to the axial outer seam of one single-piece completely folded and formed cusp and leaflet structure 730. The inner diameter D of the open frame, e.g., 19 - 35 mm naturally defines the deployed and operating diameter of the valve assembly after implantation in the body. The strut members 921 are of specific length and orientation to permit radial collapse and compression of the frame to a small diameter, e.g., 3-7 mm. The mounting bars 922 are near to equally spaced around the inner circumferential course of the frame. The length L of the arc along the inner circumference of the frame from the center of the mounting bar 922 to the center of the closest mounting bar 922 is approximately equal to (pi x D)/3. Thus defined, L also defines the transverse circumferential distance between folds 705 and 706 , approximating the circumferential extent of the portions of the joined cusp wall sections 761 and 771 extending between 705 and 706 of the folded cusp and leaflet structure of appropriate size when mounted within the frame 920.

The axial mounting bars 922 optionally contain holes and/or slots to facilitate suture attachment of the folded integrated cusp and leaflet structures 730. The frame is depicted in Figs. 9A-9E as having axial mounting bars 922 each with a hole near the proximal end to facilitate suture attachment of the apical (most proximal) portion of the folded cusp and leaflet structure.

Fig. 9B shows the metal lattice frame of Fig. 9A in the same perspective, but with the view centered on the slotted frame member 923.

Fig. 9C shows a side perspective view of the frame 920 centered on the axial mounting bar 922 with a superimposed plan view of the outer aspect of the completely folded integrated cusp and leaflet structure 730 (of Fig. 7I) as mounted within the frame 920 to demonstrate the relationships between the two. An example suture pattern for attachment is shown. The cusp wall seam 732 is aligned upon the inner surface of the mounting bar 922 of the frame 920 and attached by sutures in this example.

The commissure tabs 762 and 772 are to be understood as having been passed through the frame slots 924 from within the central space of the frame to the outer (abluminal) side and folded along 705 and 706, respectively onto the outer surface of the cusp and leaflet structure where they are attached along their common length both to the frame members 923 and, through the interspaces of the frame 920, to the radially underlying outer aspect of the cusp wall sections 761 and 771, respectively. The adjacent cusp and leaflet structures of the three-leaflet valve are not shown for clarity. The joining of adjacent commissure tabs at the slotted members 923 is demonstrated in Fig. 9D.

At the apical (most proximal) extent of the completely folded integrated cusp and leaflet structure 730, the apical portion folded radially outward along fold 703 is attached to the lower (most proximal) end of the axial mounting bar 922. When present, a hole near the end of the axial mounting bar 922 facilitates suture attachment at this point.

Fig. 9D shows a side perspective view of the frame 920 centered on the slotted frame member 923AB with a superimposed perspective view of the outer aspect of two circumferentially adjacent completely folded integrated cusp and leaflet structures 730A and 730B (of Fig. 7I) to demonstrate their relationships as mounted within the frame 920. An example suture pattern for attachment is shown. Suture attachment of the commissure folds 705 and 706 at the level of the slot is notably absent. Rather, attachment of the bodies of the commissure tabs 762A and 772B to the outer aspect of the frame at points removed from the free edges and folds of the material avoids suture penetration along the lines of traction in the slot and enhances the resistance of the structure to tearing at such suture attachments. The cusp wall seams 732A and 732B are aligned upon the inner surface of the mounting bars 922A and 922B, respectively of the frame 920 and attached by sutures in this example.

The commissure tabs 762A and 772B are to be understood as having been passed through the frame slot 924 from within the central space of the frame to the outer (abluminal) side and folded along 705A and 706B, respectively onto the outer surface of the cusp and leaflet structure where they are attached along their common length both to the frame member 923AB and, through the interspaces of the frame 920, to the radially underlying outer aspect of the cusp wall sections 761A and 771B, respectively.

Fig. 9E shows an oblique axial (top/distal) perspective view of the assembled three-leaflet valve comprising the frame 920 and three identical folded integrated cusp and leaflet structures 730A, 730B and 730C attached principally within the central space of the frame, but with the commissure tabs passed in complementary adjacent left-right pairs, 762A-772B, 762B-772C and 762C-772A, through the slots 924AB, 924BC and 924CA, of slotted frame members 923AB, 923BC, and 923CA, respectively. The view is centered on slotted member 923AB. The suture attachments are omitted for clarity.

The cusp and leaflet structure 730C farthest in view is seen within the frame 920, with the inner aspect of the seam 732C and cusp wall sections 761C and 771C in the far view. The cuff wall extension sections 718C and 728C are depicted as folded onto the outer aspect of the cusp wall sections 761C and 771C, respectively, but within the central space of the frame 920 and apposed to the inner surface of the frame. The inner (luminal) aspect of the seam 732C is shown aligned to the outwardly overlying axial mounting bar 922C to which it is attached along its length. The outer (abluminal) aspect of the top (most distal) portions of the seams 732A and 732B and the cusp wall sections 761A and 771B, of the other two folded cusp and leaflet structures 730A and 730B are also shown through the interspaces of the frame on either side of the near view.

The commissure tabs 762A and 772B, aligned and apposed along folds 705A and 706B, respectively are shown centered in the near view in position opposite to the axial mounting bar 922C and cusp wall seam 732C in the far view. The key mounting configuration of the valve commissures to the slotted frame members is here demonstrated. The triangular commissure tabs are formed as a result of the folding of the membrane template along folds 716 and 726, and are comprised of overlapping layers of the cusp wall section and the mobile leaflet section. Thus, with passage of the commissure tabs from within the interior space of the frame through the frame slots, both the cusp wall layer and the mobile leaflet layer are carried together to the outer aspect of the frame where they are attached. In addition, the interior aspect of the commissure folds 706A of cusp and leaflet structure 730A and 705B of cusp and leaflet structure 730B are shown where they mark the segment at which the commissure tabs 772A and 762B are passed through the frame slots 924CA and 924BC of slotted members 923CA and 923BC, respectively, and are tethered thereto.

The radially outward surface of the mobile leaflet sections 719A, 729A of the folded cusp and leaflet structure 730A and sections 719B, 729B of the folded cusp and leaflet structure 730B are shown on the left and right sides, respectively of the near view. (These labels omitted for clarity.)

The distal free edges of all three mobile leaflets are shown in the centrally apposed (coapted) position corresponding to the closed operating position of the valve. Fig. 9E also shows in that aspect interior to the cusps, a portion of folds 726A of cusp and leaflet structure 730A and 716B of cusp and leaflet structure 730B as they form the lower (proximal) boundary of the valve cusps.

The template examples disclosed herein are provided for enablement purposes and shall not be interpreted as limiting the scope of the claims. For example, angular values shown and/or described herein are not to be interpreted as limiting the scope of a claim unless included in a given claim.

As those skilled in the art will appreciate, circumference length varies with the diameter circumscribed therein. Accordingly, refinements in the valve manufacturing process may address adjusting the length of the leaflet free edge to be slightly less than the edge length of the cusp wall, i.e., less than the circumferential arc length between the commissures. This adjustment depends upon the dimensions of a given valve in production, as well as the dimensions of the given valve's component elements.

In still other embodiments, the percutaneously deliverable heart valve may include various other configurations by using different variations of the polygon pattern, so as to include, for example, an inner sealing cuff for the valve that is continuous and integral with the leaflet structure itself. In yet other embodiments, the percutaneously deliverable heart valve may include different configurations by adjusting the pattern and folding technique, such as the angle of the cone and its surface area, or the extent of apposition between the leaflets may also be specified.

## Claims

1. A transcatheter, implantable, prosthetic heart valve, comprising:
a frame (910, 920); and
two or more integrated cusp and leaflet folded structures (130, 530, 730) attached to the frame (910, 920), the two or more integrated cusp and leaflet folded structures (130, 530, 730) each comprising a flat sheet of biocompatible membrane that has been folded to include a mobile leaflet section (119, 129, 519, 529, 719, 729, 719A, 729A) and a cusp wall section (161, 171, 561, 571, 761, 771, 761A, 771A, 761B, 771B, 761C, 771C), wherein two non-transverse diagonally oriented folds (116, 126, 216, 226, 316, 326, 416, 426, 516, 526, 616, 626, 716, 726) are located between the mobile leaflet section (119, 129, 519, 529, 719, 729, 719A, 729A) and the cusp wall section (161, 171, 561, 571, 761, 771, 761A, 771A, 761B, 771B, 761C, 771C), wherein the cusp wall section (161, 171, 561, 571, 761, 771, 761A, 771A, 761B, 771B, 761C, 771C) is located radially outside of the mobile leaflet section (119, 129, 519, 529, 719, 729, 719A, 729A), and wherein the cusp wall section (161, 171, 561, 571, 761, 771, 761A, 771A, 761B, 771B, 761C, 771C) includes a seam (132, 532, 732) that joins apposing sides of the cusp wall section (161, 171, 561, 571, 761, 771, 761A, 771A, 761B, 771B, 761C, 771C).

2. The transcatheter, implantable, prosthetic heart valve of Claim 1, wherein the two or more integrated cusp and leaflet folded structures (130, 530, 730) are each attached along their respective seams (132, 532, 732) to the frame (910, 920).

3. The transcatheter, implantable, prosthetic heart valve of Claim 2, wherein the seams (132, 532, 732) are oriented in a direction substantially parallel to an axis of the frame (910, 920).

4. The transcatheter, implantable, prosthetic heart valve of Claim 1, wherein the flat sheet of biocompatible membrane forming at least one integrated cusp and leaflet folded structure (130, 530, 730) of the two or more integrated cusp and leaflet folded structures (130, 530, 730) comprises two or more pieces of biocompatible membrane material.

## Patentansprüche

1. Transkatheterische, implantierbare, prothetische Herzklappe, umfassend:
einen Rahmen (910, 920); und
zwei oder mehr integrierte Höcker-und-Faltblatt-Faltenstrukturen (130, 530, 730), die an dem Rahmen (910, 920) angeordnet sind, wobei die zwei oder mehr integrierten Höcker-und-Faltblatt-Faltenstrukturen (130, 530, 730) jeweils ein flaches Blatt von biokompatibler Membran aufweisen, das gefaltet ist, um einen mobilen Faltblattabschnitt (119, 129, 519, 529, 719, 729, 719A, 729A) und einen Höckerwandabschnitt (161, 171, 561, 571, 761, 771, 761A, 771A, 761B, 771B, 761C, 771C) aufzuweisen, wobei zwei nicht-quer, diagonal ausgerichtete Falten (116, 126, 216, 226, 316, 326, 416, 426, 516, 526, 616, 626, 716, 726) zwischen dem mobilen Faltblattabschnitt (119, 129, 519, 529, 719, 729, 719A, 729A) und dem Höckerwandabschnitt (161, 171, 561, 571, 761, 771, 761A, 771A, 761B, 771B, 761C, 771C) angeordnet sind, wobei der Höckerwandabschnitt (161, 171, 561, 571, 761, 771, 761A, 771A, 761B, 771B, 761C, 771C) radial außerhalb des mobilen Faltblattabschnitts (119, 129, 519, 529, 719, 729, 719A, 729A) angeordnet ist, und wobei der Höckerwandabschnitt (161, 171, 561, 571, 761, 771, 761A, 771A, 761B, 771B, 761C, 771C) eine Naht (132, 532, 732) aufweist, die aneinanderliegende Seiten des Höckerwandabschnitts (161, 171, 561, 571, 761, 771, 761A, 771A, 761B, 771B, 761C, 771C) miteinander verbindet.

2. Transkatheterische, implantierbare, prothetische Herzklappe nach Anspruch 1, wobei die zwei oder mehr integrierten Höcker-und-Faltblatt-Faltenstrukturen (130, 530, 730) jeweils entlang ihrer jeweiligen Nähte (132, 532, 732) an den Rahmen (910, 920) angeordnet sind.

3. Transkatheterische, implantierbare, prothetische Herzklappe nach Anspruch 2, wobei die Nähte (132, 532, 732) in einer Richtung im Wesentlichen parallel zu einer Achse des Rahmens (910, 920) ausgerichtet sind.

4. Transkatheterische, implantierbare, prothetische Herzklappe nach Anspruch 1, wobei das flache Blatt von biokompatibler Membran, das mindestens eine integrierte Höcker-und-Faltblatt-Faltenstrukturen (130, 530, 730) der zwei oder mehr integrierten Höcker-und-Faltblatt-Faltenstrukturen (130, 530, 730) bildet, zwei oder mehr Stücke von biokompatiblem Membranmaterial aufweist.

## Revendications

1. Valvule cardiaque prothétique implantable transcathéter, comprenant :
un cadre (910, 920) ; et
deux structures pliées de cuspides et de feuillets intégrés (130, 530, 730) ou plus fixées au cadre (910, 920), les deux structures pliées de cuspides et de feuillets intégrés (130, 530, 730) ou plus comprenant chacune une feuille plate de membrane biocompatible qui a été pliée pour comprendre une section de feuillet mobile (119, 129, 519, 529, 719, 729, 719A, 729A) et une section de paroi de cuspide (161, 171, 561, 571, 761, 771, 761A, 771A, 761B, 771B, 761C, 771C), où deux plis non transversaux orientés en diagonale (116, 126, 216, 226, 316, 326, 416, 426, 516, 526, 616, 626, 716, 726) sont situés entre la section de feuillet mobile (119, 129, 519, 529, 719, 729, 719A, 729A) et la section de paroi de cuspide (161, 171, 561, 571, 761, 771, 761A, 771A, 761B, 771B, 761C, 771C), où la section de paroi de cuspide (161, 171, 561, 571, 761, 771, 761A, 771A, 761B, 771B, 761C, 771C) est située radialement à l'extérieur de la section de feuillet mobile (119, 129, 519, 529, 719, 729, 719A, 729A), et où la section de paroi de cuspide (161, 171, 561, 571, 761, 771, 761A, 771A, 761B, 771B, 761C, 771C) comprend une jonction (132, 532, 732) qui relie des côtés opposés de la section de paroi de cuspide (161, 171, 561, 571, 761, 771, 761A, 771A, 761B, 771B, 761C, 771C).

2. Valvule cardiaque prothétique implantable transcathéter de la revendication 1, dans laquelle les deux structures pliées de cuspides et de feuillets intégrés (130, 530, 730) ou plus sont chacune fixées le long de leurs jonctions respectives (132, 532, 732) au cadre (910, 920).

3. Valvule cardiaque prothétique implantable transcathéter de la revendication 2, dans laquelle les jonctions (132, 532, 732) sont orientées dans une direction sensiblement parallèle à un axe du cadre (910, 920).

4. Valvule cardiaque prothétique implantable transcathéter de la revendication 1, dans laquelle la feuille plate de membrane biocompatible formant au moins une structure pliée de cuspides et de feuillets intégrés (130, 530, 730) des deux structures pliées de cuspides et de feuillets intégrés (130, 530, 730) ou plus comprend deux pièces ou plus de matériau de membrane biocompatible.
